(19) Europäisches Patentamt
European Patent Office
Office européen des brevets

(11) EP 2 562 690 A2

(12) EUROPEAN PATENT APPLICATION

(43) Date of publication:
27.02.2013 Bulletin 2013/09

(51) Int Cl.:
G06K 9/62 (2006.01)    G06K 9/00 (2006.01)

(21) Application number: 12170557.8

(22) Date of filing: 01.06.2012

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 22.08.2011 EP 11178290

(71) Applicant: SIEMENS AKTIENGESELLSCHAFT
80333 München (DE)

(72) Inventors:
• Costa, Maria Jimena
90482 Nuernberg (DE)
• Tsymbal, Alexey
91052 Erlangen (DE)
• Seifert, Sascha
91052 Erlangen (DE)
• Sühling, Michael
91052 Erlangen (DE)

(54) **Assigning a number of reference measurement data sets to an input measurement data set**

(57)    The invention concerns a Method and system for assigning a number of reference measurement data sets (A, R2, R3, R4, R5), selected from a plurality of reference measurement data sets (R1, ..., Ri) to an input measurement data set (I) comprising the following steps:
- determining a query instance (QD) based on the input measurement data set (I)
- providing a Random Forest (RF1, RF2, RF3)
- determining query leaves (QL1, ..., QLk) determined by the Random Forest (RF1, RF2, RF3), based on the query instance (QD)
- assigning the number of reference measurement data sets (A, R2, R3, R4, R5) to the input measurement data set (I), based on the determined query leaves (QL1, ..., QLk) and based on reference leaves (RL111, RL112, ..., RLfik) determined by the Random Forest (RF1, RF2, RF3) for a plurality of reference instances ((RD11, ..., RDfi) based on the plurality of the reference measurement data sets (R1, ...,Ri).

FIG 2

**Description**

The following invention is directed to a method for assigning a number of reference measurement data sets selected from a plurality of reference measurement data sets to an input measurement data set and a system for assigning a number of reference measurement data sets selected from a plurality of reference measurement data sets to an input measurement data set.

Experts experience, gained over many years of activity in a certain profession, provides an invaluable contribution to problem analysis and thus to support and guide related decisions. For example an engineer tends to avoid sources of defects or problems known, a physician identifies "similarities in cases" and estimates possible consequences and therapies based on his expert experience. A fundamental skill commonly attributed to experience includes the possibility to distinguish important information from less important information for reasons being unobvious at first glance, especially when important information is to be identified within vast information resources.

This is evidenced by the fact that experts are sharing their individual sources of information, and at the same time exchange their opinion about the importance of specific information, proliferating so called "expert knowledge". For example, in case of medical information, information may be shared as a compilation of files or medical reports, which combine facts and experience in one information source.

Unfortunately, the important features may vary between different contexts in which the information is to be analyzed. Moreover, information relevant for a specific context, important information or features like said "similarities" tend to be hidden or coded within less important information.

A prominent example is the analysis of medical images, in particular CT or MRT images, requiring experts experience in estimating the significance of certain features present in the image. A physician typically searches an available data base and assesses similar cases to guide his analysis and to make evidence-based decisions about patient's care (differential diagnosis, therapy selection). For example liver lesion assessment accounts for a major task in daily routine in radiology or radiotherapy.

However, even in the case of liver lesion assessment the context in which the CT images are to be analyzed may strongly vary, and thus also the important information derived from similar images or even the same identical image. Accordingly analysis might be extremely difficult and is strongly depending on the context. For example possible causes for liver lesions are numerous and varied, including both malignant (e.g. metastases) and benign (e.g. cysts or hemangiomas) pathologies. The characterization of abnormal masses constitutes an essential task on which diagnosis and the eventual treatment of the patient are based. Factors such as "size", "number", "shape", "margin definition" and "enhancement pattern" have a strong impact on the subsequent decisions. A contrast agent is often administered to the patient, and several consecutive CT scans are then acquired within some minutes. While the same lesion may look radically different in each of these consecutive CT images, two lesions originated by different pathologies that pose different risks for the patient may have almost identical appearance in some of them.

A request such as "Find lesions with comparable benignancy, in the same contrast agent phase" or "Find lesions with similar margin definition located in liver segment IVa" might be one of the tasks related to the context dependent assessment of medical images requiring fundamental expert experience.

Accordingly an object of the invention is to provide a method and a system for assigning a number of reference measurement data sets selected from a plurality of reference measurement data sets to an input measurement data set, wherein the reference data sets provide additional information for the analysis of the input measurement data set.

This object is achieved by a method for assigning a number of reference measurement data sets to an input measurement data set according to claim 1 and a system for assigning a number of reference measurement data sets to an input measurement data set according to claim 15.

According to the invention a method for assigning a number of reference measurement data sets, selected from a plurality of reference measurement data sets to an input measurement data set is provided. The term "assigning" as used in the following specification includes that a number of reference data sets are expediently noted, to be related (for example based on a ranking) to the input measurement data set. Said expedient noting may be provided for example as a list i.e. a number of data sets, like a list of patients, for the the input measurement data set, e.g. a given "case" or a medical image.

Said number, i.e. one or more, of reference data sets are selected from a plurality of reference data sets. In the following, the plurality of reference data sets may be represented by anything providing a collection of reference measurement data sets, also called a data base, a data base collection, data base samples or an archive. Said plurality of reference measurement data sets is preferably provided in an initial step of the method according to the invention. The data base preferably comprises cases or pathologies like liver lesions etc., and is even more preferably provided in a fast and reliably accessibly way, for example via an electronic data base system comprising multiple reference measurement data sets represented electronically.

The method further comprises the steps:

Preferably, providing an input measurement data set, also called input sample for search, advantageously a "case" or a patient file, comprising measurement data, as for example a medical image (i.e. 3D CT image) related to a case or a patient.

In another step, a Random Forest, trained for a classification and/or a regression problem, is provided.

It is to be noted that any of the already described commencing steps may be carried out independently, i.e. said incipient steps may be carried out in an arbitrary order.

A further step comprises determining a query instance, based on the input measurement data set, describing the input measurement data set, i.e. the input sample, for search by a set of features. Included is that the query instance may be formed of the measurement data set and/or may comprise features based on the input measurement sample. The respective set of features might be compiled as a vector, also called the feature vector.

Said given and trained Random Forest comprises a set of k decision trees (wherein k is a natural number), wherein each tree provides leaves, i.e. terminal positions or terminal nodes of the tree(also called "terminals"), indicating a classification or regression range based on the query instance. The trained Random Forest provides a model for a specific categorization or regression problem, which categorization or regression problem is in the following called "context". The "model output" or "model" yield for a specific context is based on the leaves given by the Random Forest for a respective instance (i.e. set of features as defined for example for a query instance or other instances) propagated down all k trees. The vote or yield of the Random Forest, i.e. a categorization or regression range determined, is given by the category or regression range indicated by the majority of leaves given by the Random Forest for a specific instance, respectively the query instance.

According to a subsequent step of the inventive method, query leaves, i.e. terminal nodes of the Random Forest, are determined, based on the query instance, and particularly advantageous the terminals determined or rather given by the trained Random Forest for the query instance are recorded. The recorded set of query leaves for a specific query instance is called a "query leaf set" in the following.

At this point it should be noted that an option for providing a trained Random Forest is the training of a Random Forest by incorporating "cases" or data, i.e. images. Incorporating cases or data to train a Random Forest is referenced in Breiman L., Random Forests, Machine Learning 45 (1), 2001, pp 5-32. In particular incorporating data to train a Random Forest comprises determining terminals for an instance (query instance or reference instance) describing the respective case or data, and pruning the Random Forest trees according to a known classification or regression result, given for the respective case or data as described for example in Breiman L., Random Forests, Machine Learning 45 (1), 2001, pp 5-32.

A further step in the method according to the invention comprises assigning said list, i.e. the number of reference measurement data sets, to the input measurement data set, based on the determined query leaves and based on reference leaves. Reference leaves are determined or given by the Random Forest as terminal nodes of data base samples, i.e. terminal nodes given by the Random Forest for a reference instance describing a reference measurement data set. Particularly advantageously, a reference instance is propagated down all k Trees within the given Random Forest and their terminal positions are recorded, forming a set of reference leaves of a reference measurement data set in the following referred to as "reference leaf set". The reference leaf set is hence given by the terminal positions determined for a single instance by the Random Forest. Each single reference instance is based on a single reference measurement data set of the plurality of reference measurement data sets and the reference leaves are preferably determined for each reference measurement data set comprised in the plurality of measurement data sets.

Therefore, advantageously, a method is provided which, given a new case or input measurement data set, is arranged to note and/or retrieve a list of patients, i.e. a number of reference data sets from a case data base , wherein the number of reference data sets are related to the given patient, and hence provide important information. According to the invention the relation is given by the Random Forest, and can be based on similarity criteria and/or dissimilarity criteria, as will be elaborated in more detail in the following.

The benefit of describing a categorization or classification problem using a Random Forest as a model is that query instances might be based on a variety of features. The query instance, i.e. feature vector, may have missing values concerning one or more specific features, for which the Random Forest has been trained for. Hence, appealingly the query instance is not required to encompass a mandatory set of features to provide a sufficient classification or regression result. As features, i.e. medical facts or medical evidences in the analysis of medical cases, are commonly broadly varied, the inventors have identified that Random Forests particularly support to solve a classification or regression problem in particular in the framework of medical analysis.

Moreover, it should be noted that Random Forests are a favourable model for multiclass categorization problems, as they are also customary for medical analysis tasks.

In addition, the invention fosters analysis by assigning a number of reference measurement data sets, which are all said to be relevant for the analysis of the input measurement data set in a defined way, and hence all are pertinent to a specific context. As will be shown in the following specification preferred modifications of the invention are arranged to be adapted to a variety of contexts emphasizing said benefit.

Furthermore, according to the invention a system is provided for assigning a number of reference measurement data sets selected from a plurality of reference measurement data sets to an input measurement data set.

The system comprises a query unit for determining a query instance, based on the input measurement data set. The system encompasses also a data base system for providing a plurality of reference measurement data sets. The data base system may be realized to provide any data base. The data base system may be realized preferably electronically accessible, including a link or interface, and might even more preferably be arranged to provide a plurality of reference measurement data sets on request.

The system according to the invention comprises further a Random Forest categorization unit for providing a Random Forest, which is representing a model for a specific context, and for determining query leaves of the Random Forest based on the query instance.

The system comprises also an evaluation unit for assigning the number of reference measurement data sets to the input measurement data set based on the determined query leaves and based on reference leaves determined by the Random Forest for a plurality of reference instances of the plurality of the reference measurement data sets.

Preferably the inventive system comprises also an input unit for providing the input measurement data set.

Major parts of the inventive system can preferably be realized as software running on a programmable computer system, as for example a medical image data editing station or a diagnosis station. Hence the problem is also solved by a computer program product formed to execute one or more steps of the inventive method, realizing one or more of the components of the inventive system in the form of software, arranged to be run on a respective programmable computer system. It should be noted, that the term "computer system" includes one or more Central Processing units, which might be arranged to run various components of the inventive system independently.

Particularly advantageous embodiments and features of the invention are given by the dependent claims, as revealed in the following description. Features of different claim categories may be combined as appropriate to give further embodiments not described herein.

In an advantageous modification of the invention the assignment of the number of reference measurement data sets to the input measurement data set is based on an indicator function value, which represents whether a query leaf given by the Random Forest for a query instance appears together with a reference leaf given by the (same) Random Forest for a reference instance. In other words the indicator function value represents the co-ocurrance of Random Forest leaves for two given instances, and reflects whether a terminal node of the Random Forest is comprised in both the reference leaves, i.e. the reference leaf set, and query leaves, respectively the query leaf set, or not. The co-occurrence is advantageously determined pair wise for two instances and for each terminal node given by the Random Forest for each of the two instances, wherein the pair wise determination of the co-occurrence is evaluated for leaves given in the same, identical tree of the Random Forest. The indicator function may preferably yield one of two predetermined indicator function values, wherein one value, typically "one", represents or prompts the said co-occurrence, and the other value, typically "zero", represents all other comparison results.

Advantageously in that way a possibility is given to determine a "similarity score" or "score of query leaves" of the query instance (i.e. query leaves given by the Random Forest for the query instance) which appear together with a reference leaf of a reference instance (i.e. given by the Random Forest for a reference instance) for each of the plurality of reference instances. Terminal positions recorded (i.e. the query leaf set and a reference leaf set) for two instances in each of the k trees of the Random Forest can be counted, respectively summed up, based on the indicator function. The sum or count may represent the similarity score of leaves appearing together, for two given instances. As elaborated incipiently, the categorization yield of a Random Forest given for a specific instance is based on the determined leaves for the respective instance. Accordingly the more leaves of two instances appear together the more similar is the categorization result for the two instances. Hence the more similar is the importance of the two instances for a certain context. This importance is reflected by said score of query leaves.

Furthermore in an advantageous modification the plurality of reference measurement data sets is ranked according to said determined similarity score.

Based on the given definition of the indicator function, only terminal positions being identical in the same tree of the Random Forest for both instances contribute to the sum. Hence the sum represents the frequency of co-occurrence of reference leaves and query leaves for a given reference instance and a given query instance. The score is a measure for similarity of two instances and hence a favourable measure to assign a number of reference measurement data sets comprised in a plurality of reference measurement data sets to an input measurement data set. The plurality of reference measurement data sets (i.e. the data base samples) can thus be ranked according to the frequency (i.e. score or count) of co-occurrence with the input sample (i.e. respective query leaves given by the Random Forest for the query instance, describing or rather characterizing the input measurement data set). Based on said ranking the system is preferably arranged to retrieve a ranked list of patients from the data base, preferably a "case data base", that are most "similar" to the given patient or respectively the given case (input measurement data set).

An alternative for ranking the plurality of reference data sets, still based on the score or count of indicator function values, indicating for a respective reference measurement data set that query leaves given for the query instance appear together with respective reference leaves given for the respective reference measurement data set by the Random Forest is provided in a preferred modification. Accordingly, for each query leaf a list of reference data sets is determined. The determined list comprises reference data sets, for which reference leaves have been determined by the Random Forest, which are identical to the respective query leaf. The reference leaves are given by the Random Forest based on reference instances, which describe or reflect the associated reference measurement data set. Hence, the indicator function for a specific query leaf indicates or prompts that said leaf is comprised in the reference leaves for a respective reference instance for each reference measurement data set being comprised in the respective list. Respectively, for a specific query leaf comprised in a given query leaf set for a given query instance, a list of data base samples (reference measurement data sets), called a "leaf similarity list" in the following, is determined. The items of said leaf similarity list are formed of data base samples, i.e. reference measurement data sets, for which a reference leaf identically to said specific query leaf is given by the Random Forest in the same tree (i.e. given by the Random Forest for the reference instance related to said reference measurement data set).

Alternatively or additionally, the items comprised in the leaf similarity list might be formed of pointers or indexes referencing reference measurement data sets, i.e. data base samples. That includes for example file numbers or file names, i.e. data set numbers, names or other indexes identifying specific reference measurement data sets in the plurality of reference measurement data sets, i.e. in the data base or archive.

Advantageously said leaf similarity lists, which are determined for each query leaf, are combined to a combined list, and the number of occurrences of each reference data set (or indexes or pointers) in the combined list is determined, and so a frequency or count of occurrences is given.

Since the number of duplicates (i.e the count of occurrences) for each specific reference measurement data set comprised in the combined list represents the frequency of co-occurring query leaves with reference leaves given for the specific reference measurement data set by the Random Forest, the count of duplicates reflects the frequency of co-occurrence of reference leaves and query leaves. Hence a favourable way is provided for determining said score of query leaves, as defined before.

Accordingly the number of reference data sets advantageously can be ranked according to said count of occurrences, i.e. the frequency of co-occurring leaves, representing said score of query leaves, to expediently select a number of reference measurement data sets from a plurality of reference measurement data sets, i.e. assign a number of reference measurement data sets to an input measurement data set.

At this point it should be noted, that preparing a combined list and ranking the number of reference measurement data sets according to the count of occurrences in said combined list is also favourable in view of a modification of the invention combining an arbitrary number of contexts, as elaborated in the following.

Preferably the score of query leaves is normalized by the number k of Random Forest trees, yielding a so called similarity S for two instances, respectively a query instance QD and a reference instance RD1, given by

$$S(QD,RD1) = \frac{1}{k}\sum_{t=1}^{k} IF(RLt = QLt). \qquad (1)$$

Here, RLt represents the reference leaf given by a Random Forest RF1 in the tree t for the reference instance RD1, wherein the Random Forest RF1, as explained before, comprises k trees. Accordingly QLt is the reference leaf given by a Random Forest RF1 in the tree t for the query instance QD. Furthermore IF denotes the indicator function, as defined and explained before. In addition it is to be explicitly noted that equation (1) depicts a pair wise comparison of reference leaves and query leaves, and displays also summing up the yield of the pair wise comparison, given by the indicator function, representing the similarity score respectively the score of query leaves as defined above.

Normalizing the score with the number k of trees (i.e. dividing the score with k) yields the portion or fraction of the trees, in which two instances appear together in the same leaves, in particular the portion or proportion of the trees of the Random Forest, in which query leaves of a query instance (respectively given for a query instance) appear together with reference leaves given by the Random Forest for a reference instance. Hence an assigning methodology based on the Random Forest similarity is proposed.

Advantageously the number of reference measurement data sets is assigned based on the portion or proportion of the trees of the Random Forest, in which query leaves given for a query instance by the Random Forest appear together with reference leaves given by the reference forest for a reference instance determined for each reference data set comprised in the plurality of reference data sets.

With said normalization based on the number of Random Forest trees, preferably a normalization to a normalization target value of "one" (i.e. the maximum portion of trees), a defined similarity measure can be gained being independent of the number of Random Forest trees, hence providing the possibility to be compared for an arbitrary number of contexts. Each of the contexts can be represented by a respective Random Forest, and the number of Random Forest trees required to model a specific context can be chosen individually for each context. However the invention does not exclude defining a general number of trees used for all Random Forests of the arbitrary number of contexts used, for example in the range of hundred trees.

A preferred modification of the invention includes determining a so called distance D between two instances (i.e. a query instance and a reference instance). Said distance D is preferably given by

$$D(QD,RD1) = 1 - \sqrt{1 - S(QD,RD1)}, \qquad (2)$$

wherein QD represents a query instance, RD1 a reference instance and S the similarity as elaborated above.

In other words, in a preferred embodiment of the invention for each reference data set comprised in the plurality of reference data sets the square root of a normalization target value (for instance "one" as explained before) minus the normalized score of query leaves (with a maximum of "one") is determined, representing the so called distance D. It is to be noted, that the distance D is also based on said similarity S, and a respective preferred embodiment introduces a distance based framework, based on the Random Forest similarity.

Accordingly the reference data sets can be ranked based on the determined distance D (i.e. also a similarity criterion), respectively forming a "dissimilarity list". In this case, the ranking of the number of reference data sets is given in an order reflecting the least similarity S to the input measurement data set. Example applications comprise clustering analysis as the assessment of common discriminating factors of measurement data sets and might be helpful in checks for "benignancy" or "lesion type" as will be explained in more detail.. Furthermore a dissimilarity based assessment may also support multi-dimensional scaling contexts.

In a modification of the invention the input measurement data set comprises medical image data, in the following also named "volumes", and preferably comprises an X-ray image, a CT image, a MRT image, an IR radiation image, a sonographic image and/or a radionuclide emission based image, preferably a 3D image, more preferably a 3D CT scan.

Advantageously the plurality of reference data sets comprises X-ray images, CT images, MRT images, IR radiation images, sonographic images and/or radionuclide emission based images, including an arbitrary combination of said image types, and preferably also 3D images, even more preferably 3D CT scans, respectively 3D CT hepatic pathologies. The 3D images can be represented by three 2D images or rather cuts, orthogonal to each other, intersecting at a common center, preferably the center of a lesion.

As elaborated before, appealingly the Random Forest is adapted to handle a variety of feature vectors or query instances, including the ability to cope with missing values. The inventors have realized that Random Forests foster the inclusion of a plurality of data or image sources, i.e. measurement data sources. Accordingly, in an advanced modification of the invention the query instance comprises features counted or calculated from an image section and/or from the full image. Hence the diversity of features of the input measurement data set is increased, representing an additional measurement data source.

As is explained before, the Random Forest is trained for a specific context. In order to increase the possibilities, i.e. criteria, for searching and comparing aided by the Random Forest, and hence the meaningfulness of the assigned number of reference measurement data sets, an increased diversity of features of the input measurement data set is beneficial. In particular categorization or regression accuracy can be improved, which is readily identified as a meaningful result determined by the Random Forest.

Advantageously the query instance comprises a set of low level data, wherein low level data is formed of low level features, which can be counted or calculated from the input measurement data set. Hence the low level features can be advantageously computer generated. As elucidated before, the number of criteria for searching and comparing can be increased and hence a more meaningful result is to be expected.

Preferably low level data based on an input measurement dataset formed of or comprising image data can be selected as one or more items from the following group:

The group comprises histograms, i. e. a representation or display of relative frequencies, of an intensity value of radiation used to acquire the image, which are respectively counted or calculated from a section of the image, i.e. from image section data. The intensity values are preferably Hounsfield Units (HU), and the image section data is formed of i.e. a bounding box defined in the full image data, preferably for a lesion, reflecting a region of interest (ROI).

Furthermore, the group includes also four central moments, which are formed of the expected value, the variance, the skewness and the kurtosis, calculated on said histogram.

In addition the group also comprises the four first normalized central moments, formed of the expected value, variance, skewness and kurtosis based intensity values given in the order of their appearance in the image data (i.e. a intensity vector, preferably given in Hounsfield Units), hence counted or calculated solely from image section data.

Also, the group comprises eight image moment invariants defined by Hu as disclosed in M.K. Hu, Visual pattern recognition by moment in- variants, IRE Trans. Inf. Theory IT-8, 179-187 (1962), up to the $3^{rd}$ order, calculated from image section data, and/or six image moment invariants defined by Zernike up to the $3^{rd}$ order calculated solely from image section data. The Zernike image moment invariants are described in Pejnovic, P., Buturovic, L.J., Stojiljkovic, Z., Object recognition by invariants,ICPR92(II:434-437).

Moreover the group comprises an intensitiy histogram (i.e. a histogram of Hounsfield Units) of the full image, and four first normalized central moments (formed of an expected value, variance, skewness and kurtosis) calculated based said intensity histogram counted or calculated from the full image data.

In addition the group comprises the four first normalized central moments, formed of the expected value, variance, skewness and kurtosis based intensity values given in the order of their appearance in the image data (intensity vector, preferably given in Hounsfield Units), counted or calculated from the full image data.

Respectively each input sample for search (i.e. the input measurement data set) is described by a set of low-level computer-generated imaging features. The first four features or feature types of the group describe the image section data, i.e. are solely pertinent to image section data, comprising for example a lesion, preferably a liver lesion, while the last two describe the whole image, hence advantageously the whole liver.

A preferred modification of the invention includes the step of determining a query instance comprising a set of high level data, wherein high level data is formed of high level features, also called "semantic labels" or "semantic high-level features", which preferably can be comprised in or for example extracted from clinical reports. In particular high level features are distinct from low level data, which are counted or calculated from a measurement data set.

Advantageously, high level features are chosen from a set of predefined features, preferably predefined for a specific context. Hence, high level features can be chosen from a set of experts opinion based features, as they can be extracted from a clinical report. High level features include for example a contrast agent phase or a lesion focality. Accordingly, the diversity of features provided to the Random Forest can be increased and the accuracy of the prediction result is anticipated to be improved. Said benefit is particularly favourable when two instances, a query instance representing an input measurement data set based on a set of features comprising high level data and preferably low level data, and a reference instance representing a reference measurement data set based on a set of features comprising high level data and preferably low level data, are to be compared. I.e. a discrimination accuracy of a Random Forest based similarity or distance is to be evaluated.

Accordingly, a query instance comprising said high level data can be particularly also useful in a method, wherein a single reference measurement data set selected from a plurality of measurement data sets is assigned to an input measurement data set. The reference measurement data set may be assigned to the input measurement data set based on determined query leaves given by the Random Forest for the query instance and based on reference leaves determined by the Random Forest for the reference measurement data set, i.e. given by the Random Forest for a reference instances representing the reference measurement data set, on the basis of high level features and preferably low level features.

In a preferred embodiment of the invention high level data based on the input measurement dataset, which is formed of or comprises image data, are selected as one or more items from a group of features.

This group of high level features may comprise a "Contrast agent phase" wherein possible feature values may be chosen from a set of values comprising "Native", "Arterial", "Portal Venous", "Late". The value "Native" reflects the absence of contrast agent, the value "Arterial" an arterial contrast agent phase, the value "Portal Venous" a Portal Venous contrast agent phase and the value, "Late" a late contrast agent phase at the moment of acquisition of the image or input measurement data set.

Furthermore, the group can comprise a high level feature "Lesion focality" wherein possible feature values may be chosen from a set of values comprising "Single" and "Multiple". The value "Single" reflects a single lesion of the given type comprised in the respective image or region of the patients body and the value "Multiple" reflects more than one lesions of the given type within the respective image or region of the patients body.

Moreover, the group comprises a high level feature "Lesion surrounding" wherein possible feature values may be chosen from a set of values comprising "Complete", "Incomplete" and "Absent". The value "Incomplete" reflects a lesion, wherein parts of the surroundings are discontinuous or missing, the value "Complete" a lesion, wherein there are no discontinuities or missing portions of the surroundings, and the value, "Absent" a lesion wherein there are no visible surroundings.

The group of high level features can also comprise as a high level feature "Rim Continuity", wherein possible feature values may be chosen from a set of values comprising "Contious_Bright_Rim", "Discontinous_Bright_Rim", "Continous_Dark_Rim", "Discontinous_Dark_Rim". The said values reflect a lesion, wherein "Continuous" represents an uninterrupted rim, opposing to "Discontinous" in which the rim may present discontinuities or missing portions. The terms "Bright" and "Dark" represent the relative intensity of the rim with respect to that of the surrounding structure's expected intensity (e.g. with respect to the intensity range of the normal surrounding liver parenchyma).

An additional high level feature "Margin" may be also comprised in the group, wherein possible feature values may be chosen from a set of values comprising "Regular" and "Irregular". The value "Regular" represents a lesion with a smooth, continuous margin, in contrast to a lesion with rugged, unsmooth, discontinuous margin being represented by a value "Irregular".

Moreover, the feature "Margin definition" may be also comprised in the group, wherein possible feature values may be chosen from a set of values comprising "Defined" and "Diffuse". The value "Defined" in this case represents a clear demarcation of what represents the inside and the outside of the lesion in question. In contrast, a lesion with a fuzzy or unclear demarcation of its boundaries (i.e. what is the "outside" and what is "inside" of the aforementioned lesion) is reflected by the value "Diffuse".

The features chosen from said group of high level features are particularly favourably in describing liver lesions, however any other type of lesion may be characterized by said group of high level features as well. And in particular it has to be noted that such a "high-level concept" based on low level data and on high level data, incorporating features which can not be calculated or measured from a measurement data set (i.e. input measurement data set or reference measurement data set) into a feature vector or instance (i.e. query instance or reference instance) may improve the prediction accuracy for any given context independent of analysis of lesions.

Preferably the leaves (terminal nodes) determined by the Random Forest are provided as a set of leaves (i.e. their terminal positions z in each of the trees are recorded in said query leaf set and said reference leaf set), wherein a leaf index vector comprising k terminal nodes (i.e. the number of trees of a trained Random Forest for a given context) is formed by said set of leaves in a given order. In other words, a leaf given by a specific tree of the Random Forest is identifiable within the set of leaves representing a leaf index vector. Preferably the leaf index vector is assigned to data sets comprised in a data base system , i.e. the plurality of reference measurement data sets and, advantageously, reference measurement data sets comprised in the data base system are indexed according to said provided leaf index vector. Accordingly, the data base system is arranged to identify an assigned or categorized reference measurement data set based on the leaf index vector. For an electronic data base system comprising a plurality of image data sets representing reference measurement data sets, the term "indexing" implies that for each image data set an assigned leaf index vector is stored in the electronic data base, and the data base can be searched, based on a leaf index vector. Hence retrieval of image or measurement data sets is preferably based on the leaf index vector. Furthermore, since the leaf index vector comprises a "type classification information" or "regression information" for a specific context, the retrieval of similar cases and/or the retrieval of a type classification is possible.

Favourably for an arbitrary number of contexts which can be applied and easily combined as search criteria in a preferred embodiment or framework of the invention, the data base system is preferably arranged to assign a plurality of leaf index vectors to the reference data sets. Preferably a plurality of leaf index vectors is associated with a specific reference data set and stored in the electronic data base system, wherein the number of storage possibilities, i.e. data base items, data base entries, data base fields or field identifiers etc., for leaf index vectors associated with a specific reference measurement data set preferably is at least as large as the number of available trained Random Forests.

Furthermore, increasing the plurality of reference measurement data sets available for analysis, according to a preferred embodiment, the input measurement data set is categorized according to the determined query leaves. I.e. a type categorization is determined by the Random Forest, given by the determined query leaves, and the input measurement data set is stored in the data base system and indexed based on the determined query leaves.

Preferably, the provided Random Forest is retrained in predetermined intervals, hence repeatedly, based on a predetermined condition. For instance the Random Forest can be retrained by incorporating the new cases at a regular basis, i.e. the predetermined condition is a time intervall. However it is also conceivable that the predetermined condition includes the condition that a predetermined number of new cases or input measurement data sets is available and was preferably categorized by the random forest. Moreover the predetermined condition may also include that a predetermined number of new features are attributed for the specific context represented by the random forest to be trained ("new feature" in this case means that the query instances which were used to train the Random Forest, did not include the respective new features at the time of training).

Other objects and features of the present invention will become apparent from the following detailed descriptions considered in conjunction with the accompanying drawings. It is to be understood, however, that the drawings are designed solely for the purposes of illustration and not as a definition of the limits of the invention. The drawings show in

Figure 1 a schematic overview of an embodiment of the invention, wherein, based on similarity to a new case, a number of images are retrieved from a case data base;

Figure 2 a flowchart of the method used in the embodiment of Figure 1 in more detail, explaining a decision step;

Figure 3 a flowchart of a method for realizing the decision step of Figure 2;

Figure 4 a flowchart of an alternative method for realizing the decision step of Figure 2;

Figure 5 a flowchart of another alternative method for realizing the decision step of Figure 2;

Figure 6 a schematic overview of the determination of a query instance;

Figure 7 a list of reference measurement data sets determined by the invention;

Figure 8 a schematic overview of an embodiment of the invention;

Figure 9 a schematic overview of a flowchart of an embodiment of a method according to the invention;

Figure 10 a schematic overview of an embodiment of a system according to the invention;

Figure 11 evalution results, showing the relevance of the assigned number of reference measurement data sets for the input measurement data set; and

Figure 12 images of lesions outlining the problem of the invention.

Although the invention is described mainly by embodiments representing a so called Content Based Image Retrieval (CBIR) system to support clinical decisions, such embodiments are solely illustrative and are not intended to limit the invention.

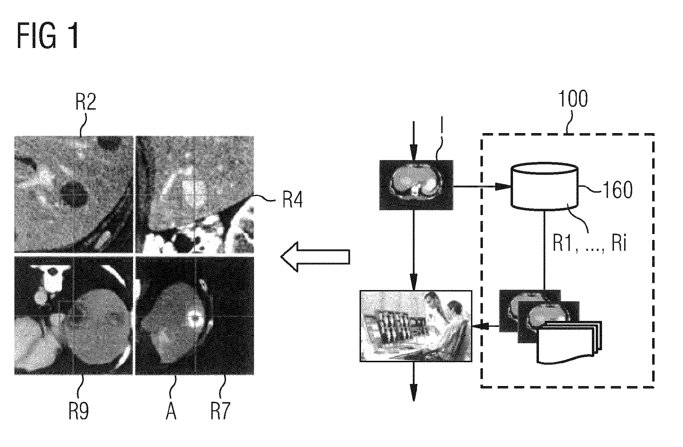

In particular in Figure 1 a clinical decision support system 100 is displayed that helps physicians (Radiologists) to make evidence-based decisions about patient's care (differential diagnosis, therapy selection). The concrete example application concerns liver lesion assessment which accounts for a major task in daily routine in radiology or radiotherapy.

Possible causes for liver lesions are numerous and varied, including both malignant (e.g. metastases) and benign (e.g. cysts or hemangiomas) pathologies. The characterization of abnormal masses constitutes an essential task on which diagnosis and the eventual treatment of the patient are based. Factors such as size, number, shape, margin definition and enhancement pattern have a strong impact on the subsequent decisions. A contrast agent is often administered to the patient, and several consecutive CT scans are then acquired within some minutes. While the same lesion may look radically different in each of these consecutive CT images, two lesions originated by different pathologies that pose different risks for the patient may have almost identical appearance in some of them.

As will be shown, in a preferred embodiment, considerably more relevant results can be produced if the retrieval framework is able to respond to user requests such as "Find lesions with comparable benignancy, in the same contrast agent phase" or "Find lesions with similar margin definition located in liver segment IVa". Towards this goal, according to a preferred embodiment as shown in Figure 1, a fast, flexible, learning-based Content Based Image Retrieval (CBIR) framework is introduced that can accommodate combinations of low-level features and user-defined similarity criteria and concepts in order to retrieve, i.e. assign, the lesions most relevant in the given clinical context. Furthermore, once the learning phase has been completed, the framework can function independent of high-level annotations of the data base lesions, making the processing of new volumes fast and simple.

While similar lesion retrieval can be a powerful decision support tool for clinicians, as well as an invaluable learning support for students, if the criteria for searching and comparing lesions is based solely on low-level features, the clinical meaningfulness of the retrieval results can be improved by considering high level features present in clinical reports, as will be shown in a preferred embodiment. In other words, the invention provides the possibility to retrieve a number of measurement data sets from a plurality of reference measurement sets based on relevance criteria pertinent to a specific context.

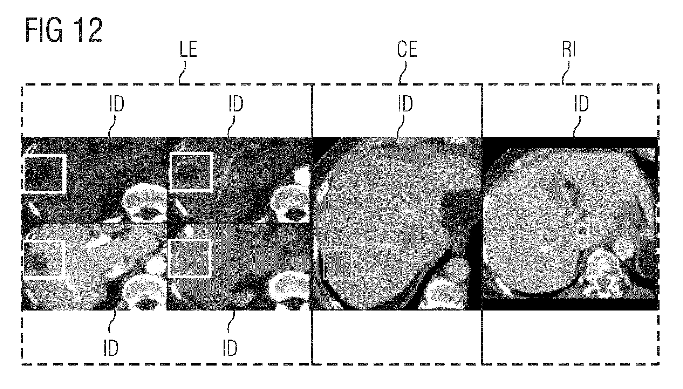

The problem tackled by the preferred embodiment of figure 1 is illustrated in more detail in Figure 12. The leftmost image LE, comprising liver CT image data ID, shows the varying appearance of the same lesion in four different contrast agent phases. At the center CE and right RI, two lesions with similar appearance are shown, even though the one in the center CE is malignant (metastasis) and the rightmost RI one is benign (cyst).

In the cases shown, a search for similar lesions based only on low-level criteria would likely suggest that the first lesion (shown in the four pictures at the left LI) is not similar to itself, while the two lesions shown in the pictures in middle (center CE) and on the right RI side would probably be retrieved as similar even though their benignancy and the patient outlook and treatment in each case would be very different.

In Figure 1 a CBIR system 100 is presented, which is based on a learning-based method for fast content-based retrieval of similar 3D images.

The system 100, is arranged to assign, similar 3D CT images to a new (as indicated by an arrow) input case represented by image data. As shown, the image data is a clinical CT image, and hence is based on a clinical measurement and representing an input measurement data set I to the system 100. Accordingly the system 100 can be called "assignment system". The system 100 comprises a data base system 160, for archiving a plurality of 3D images, i.e.

providing a plurality of reference measurement data sets. The data base system 160 can be automatically queried based on the input measurement data set I, i.e. based on a new input case for a number of similar 3D images. Accordingly the data base system 160 can be automatically queried for a number of reference measurement data sets R2, R4, R7, R9, which are to be assessed for a differential diagnosis or targeted therapy, based on an embodiment of the method according to the invention. The group of assigned reference measurement data sets will be denoted by A in the following. Hence, the system 100 is realized to associate or assign a number of reference data sets A, selected from a plurality of reference data sets R1, ..., Ri, to the new case or image. It is to be noted that in this case the index i represents the number of reference measurement data sets assessed based on the method according to the invention, and is usually identical with the number of reference measurement data sets available in the data base 160. As will be elaborated in more detail in the following, other than retrieving a number of similar images the system 100 is also realized to perform a type classification of the new case or image data and also for the plurality of the cases or reference measurement data sets R1,..., Ri present in the data base system 160. As will become more apparent in the following, adaption to a specific analysis problem i.e. a context is possible - the ability to learn is gained.

As is also shown, retrieving similar cases in the preferred embodiment of figure 1 includes the retrieval of clinical reports also present in the archive. Alternatively, it is likewise conceivable that the reports can be assigned to the reference measurement data sets R1,..., Ri at the time of retrieval, hence they can be comprised in another data base system independent of the data base system 160 comprising the plurality of images (i.e. reference measurement data sets R1, ..., Ri). Related clinical reports comprise additional information relevant for the reference measurement data set R1,..., Ri or image, and provide features based on an experts opinion, which can not be counted or calculated from the reference measurement data set R1, ..., Ri or image directly. Based on this additional information a clinical meaningful assessment of the number of images retrieved from the archive is supported.

In particular, as will be elaborated in more detail, in the framework of the invention the possibility of the combination of flexible user-defined contexts and high-level concepts with low-level features can be provided in order to yield search results, i.e. assigned number of reference measurement data sets A, that are both meaningful to the user and relevant in the given context.

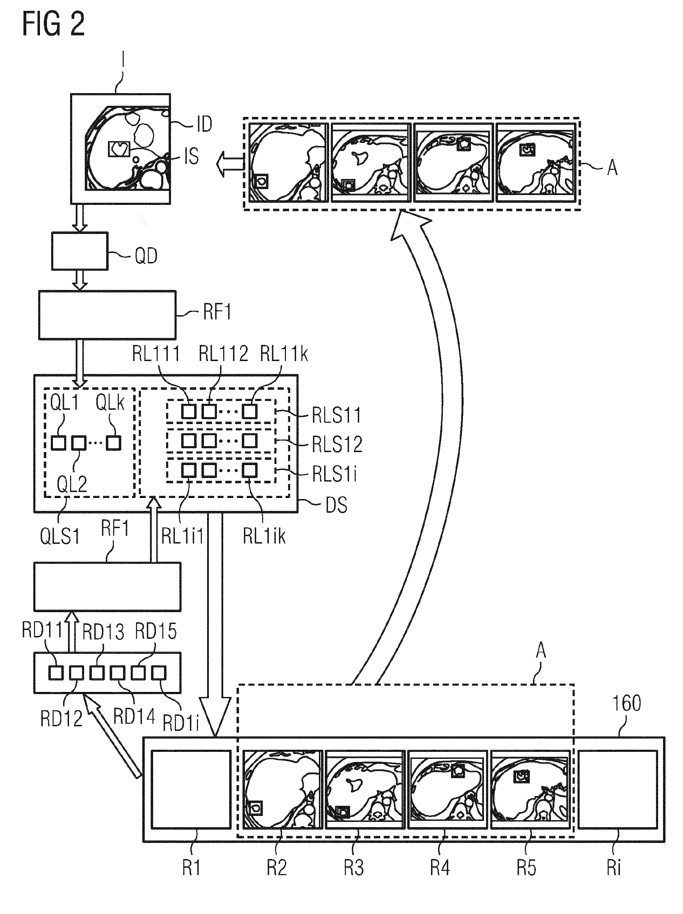

A preferred embodiment of the invention, for example as outlined in Figure 2, realizes a fast distance learning-based CBIR framework based on the intrinsic Random Forest similarity.

Figure 2 shows a schematic overview of an flowchart of such an embodiment of the method to assign a number of reference measurement data sets A selected from a plurality of reference measurement data sets R1, ..., Ri comprised in the data base system 160 . Here, a new case or input measurement data set I is presented to the system as outlined in regard of Figure 1. Due to the complexity and clinical relevance of the domain (i.e. the vast application possibilities of the invention) the invention is particularly illustrated in an embodiment in which the invention is applied to the retrieval of similar 3D CT hepatic pathologies.

According to said embodiment, the input measurement data set I comprises image data ID, including a liver lesion, embedded in a bounding box or region of interest (ROI), which reflects a section IS of the image data ID.

In an initial step, based on the input measurement data set I, a query instance QD is determined, comprising a set of features based on the input measurement data set I, previously defined as feature vector. As will be further elaborated, the feature vector can comprise features derived from the image data ID as for example a Hounsfield Unit distribution in the case of CT images or other features important to the respective case or input measurement data set I. The query instance QD is presented to a Random Forest RF1 formed of k Random Forest trees. In the present embodiment directed to liver lesion analysis, k is in the range of 100, which has proven to be particularly favourable for the context of retrieval of similar 3D CT hepatic pathologies. The Random Forest RF1 has been trained for the categorization according to a benignancy to the lesion comprised in the bounding box in order to retrieve similar images with comparable benignancy to the lesion comprised in the bounding box or image section IS. Hence the Random Forest RF1 has been trained for said specific, given context. The query instance QD is propagated down all k trees within the Random Forest RF1 and the terminal position or query leaf QL1, ..., QLk in each of the k trees are recorded, (i.e. the notation for a query leaf is QLt with t=1, ..., k) .

Before, in parallel or subsequently to said initial step, for each reference measurement data set R1, ..., Ri comprised in the data base system 160, a reference instance given by the notation RDfi is determined. Each of the reference instances RDfi comprises a set of features, based on the respective reference measurement data set R1, ..., Ri. The notation RDfi is chosen such that the index f denotes a specific random forest, here the Random Forest RF1 (i.e. f=1), and the second index i denotes a specific reference measurement data set R1, ..., Ri described by the reference instance RD11, ..., RDfi. The reference instances RD11, RD11, ..., RD1i are presented to the Random Forest RF1 (it is to be noted that for the given random forest RF1, only suitable instances RD11, ..., RD1i are fed to the respective random forest, i.e. reference instances pertinent to the specific context modelled by the Random Forest RF1 and indexed with f = 1), and also the reference instances RD11, ..., RD1i are propagated down all k trees within the Random Forest RF1 and the terminal positions or the reference leaves denoted by RLfit in each of the k trees are recorded. The index f represents a particular Random Forest as mentioned above. In the present embodiment of figure 1 the index f is fixed to 1 pointing to the available Random Forest RF1. However, as will be elaborated, it is conceivable that a plurality of Random Forest are used in the inventive method, hence index f can deviate from 1. Index i denotes the respective measurement data set R1, ...,Ri and t = 1, ..., k represents the $t^{th}$ tree of the Random Forest RF1.

In a decision step DS, based on the determined reference leaves RL111, ..., RLlik (k is the number of Random Forest trees of Random Forest RF1, hence k is the maximum of t) and the determined query leaves QL1, ..., QLk a number of reference measurement data sets A is selected from the plurality of reference measurement data sets R1, ..., Ri comprised in the data base system 160 and assigned to the input measurement data set I. In the present case a number of images with comparable benignancy to the lesion comprised in the bounding box are retrieved from the data base system 160 and assigned to the image or to the new case which is due to be assessed.

Other contexts which can be modelled each by an independent Random Forests in the framework of liver lesion analysis include the analysis of the lesion density (i.e. Hypodense, Hyperdense) or the lesion type (Cyst, HCC, Hemangioma, Metastasis).

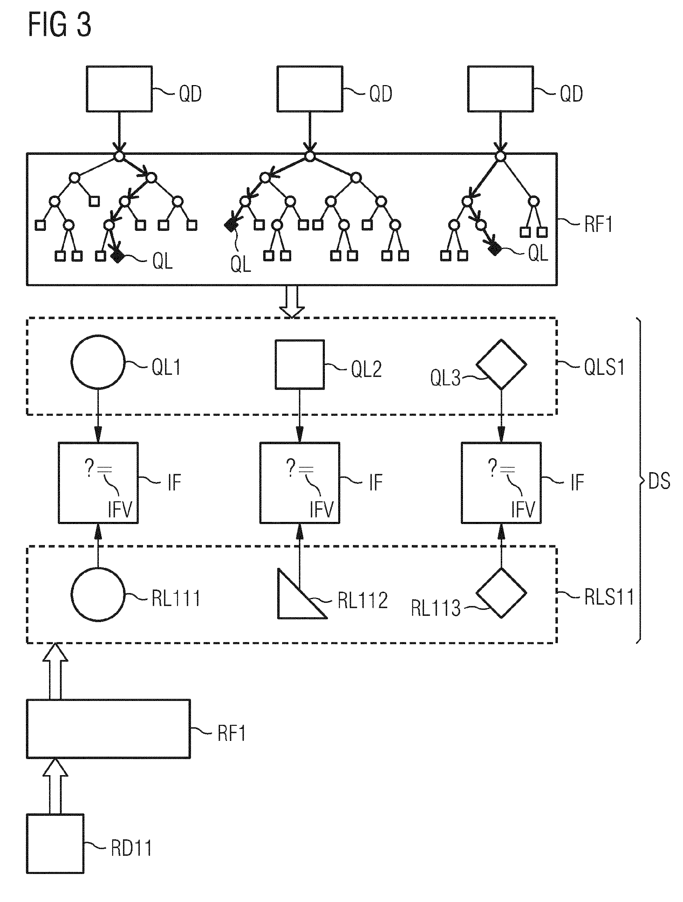

Figure 3 illustrates the decision step DS, wherein here, for the sake of simplification, only three decision trees of the Random Forest RF1 are shown. It is to be understood that the Random Forest RF1 of Figure 3 may be identical to the Random Forest RF1 of Figure 2, and hence comprises around 100 forest trees.

For a Random Forest RF1 trained for a certain classification problem, the proportion of the trees where two instances appear together in the same leaves can be used as a measure of similarity between them.

For a given forest RF1 the similarity between two instances is calculated as follows:

The instances are propagated down all k trees within RF1 and their terminal positions in each of the trees are recorded. In the present case similarity between the input measurement data set and a reference measurement data set is to be evaluated. Hence the two instances which are run down all k trees are the query instance QD and a respective reference instance RD11 representing its associated reference measurement set comprised in the data base.

For the particular detail of Figure 3 (with only three trees in the random forest RF1), the Random Forest RF1 yields for the query instance QD three query leaves QL1, QL2, QL3. QL1 is represented by a circle, indicating a particular category for example "rather_benign" in the first Random Forest tree of the Random Forest RF1. QL2, illustrated by a square, indicates also a particular category accordingly in the second tree, in the present case also the category "rather_ benign". Moreover, QL3, pictured as a diamond, represents a particular category in the third tree of the Random Forest RF1, in the given case the category "rather_malign". Exceeding the illustrated detail, the given query leaves QL1, QL2, QL3 for all k Random Forest trees form the query leaf set QLS1 for the particular query instance QD, hence the query leaf set QLS1 comprises k query leaves or respectively k representations of categories for a particular context. The notation QLSf (here f=1) is again chosen such, that index f points to a particular Random Forest; the Random Forest RF1 in the present case. Hence for every Random Forest RF1 used, a specific query leaf set QLSf is determined based on the same query instance QD. A vote of the Random Forest RF1 for a particular category determined for the input measurement data set represented by the query instance QD is determined by the category, which is given most frequently by the Random Forest trees of the particular Random Forest RF1.

Furthermore the said three trees of the Random Forest RF1 give the reference leaves RL111, RL112 and RL113 for a particular reference instance RD11, comprising features which are relevant for a reference measurement data set comprised in the data base system. A reference leaf RL111 identified (indicating a categorization or regression result) in the first tree of the Random Forest RF1 for the reference instance RD11 is identified by a circle and represents as mentioned above the category "rather_benign" in the first tree. A second reference leaf RL112, identified by a triangle, indicates a category "benign" in the second tree of the Random Forest RF1. And a third reference leaf RL113 given in a third three of the Random Forest RF1, represented by a diamond, indicates a category "rather_malign", as also explained before. Analogously to the case of a query leaf set QLS1, the given reference leaves RL111, RL112, RL113 for all k Random Forest trees form a reference leaf set RLS11 for the particular reference instance RD11.

The similarity S between the two instances, respectively between the query instance QD and the reference instance RD11, is based on the evaluation of the indicator function IF and equals to the equation (1), incipiently explained, which is given here again using the notation of the explicit embodiment of figure 3:

$$S(QD,RD11) = \frac{1}{k}\sum_{t=1}^{k} IF(RL11t = QLt), \qquad (1a)$$

wherein k again represents the total number of Random Forest trees in the Random forest and t = 1, ..., k is the number or index of the tree. It is to be understood that, preferably, all k trees of the Random Forest RF1 are considered in the computation of the Random Forest similarity S.

Referring again to Figure 3, a pair wise evaluation of the indicator function IF for a reference leaf RL111, RL112, RL113 and a query leaf given QL1, QL2, QL3 for a particular tree of the Random Forest RF1 as demonstrated in equation (1a) yields for reference leaf RL111 and the query leaf QL1, which are both given in the first tree, a result of "one". The indicator function value IFV "one" indicates that both the query leaf QL1 and the reference leaf RL111 represent the same category, symbolized by said circle in the first tree. Hence the value of "one" indicates the co-occurence of a particular category, in the present case "rather_benign" represented by a particular leaf for two given instances (i.e. the query instance QD and the reference instance RD11) in a particular tree of the Random Forest RF1.

Furthermore the indicator function IF yields a value of "zero" for the pair wise evaluation of query leaf QL2 and reference leaf RL112, both given in the second tree of the Random Forest RF1. Said leaves QL2, RL112 symbolized by a square and a triangle indicate diverging categories, and hence the value of "zero" prompts different categories for the two instances QD, RD11 given in the particular tree of the Random Forest RF1. Accordingly, the indicator function value IFV of "zero" accounts for lacking co-occurrence of leaves QL2, RL112 given for two instances (the query instance QD and the reference instance RD11) in a particular tree.

On the other hand, as the diamonds determined for QL3 and RL113 represent an identical category or terminal position in the particular third tree of the Random Forest RF1 for a given instance (i.e. the query instance QD and the reference instance RD11) it can be readily perceived that the indicator function IF yields a value IFV of "one" for the pair wise comparison of the query leaf QL3 and reference leaf RL113.

Hence the sum of indicator function values IFV as given by equation (1a) which represents a "score" or "similarity score" for said two instances QD and RD11 yields a value of "two" for the three Random Forest trees. The proportion of the trees where two instances appear together in the same leaves is given by dividing the sum of indicator function values or the score, by the total number of trees in the Random Forest. For the example with three Random Forest trees as shown in Figure 3 this proportion or fraction is 2/3. When dissimilarity or distance D is needed and not a similarity (e.g., for clustering or multi-dimensional scaling) it is normally calculated for a given query instance QD and a given reference instance RD11 according to the incipiently explained equation (2), which reads for the special case of Figure 3 as follows:

$$D(QD,RD11) = 1 - \sqrt{1 - S(QD,RD11)}, \qquad (2a)$$

Thanks to the appealing properties of Random Forests, the Random Forest similarity S (cf. equation 1 or 1a) can be easily calculated for different tasks, including classification and regression problems, tasks with heterogeneous feature vectors with possible missing values, and multiclass tasks.

Both measures, the similarity S and the distance D (which is also based on the similarity S as reflected by eq. (2) or (2a)), represent a favourable measure for ranking reference measurement data sets, which are to be assigned to an input measurement data set. In the preferred embodiment of Figure 3 the pair wise comparison of query leaves QI1, QL2, QL3 with reference leaves RL111, RL112, RL113 given for the input measurement data set and a reference measurement data set, is repeated iteratively for each reference measurement data set comprised in the plurality of reference measurement data sets.

As briefly mentioned above, for both measures - the similarity S and the Distance D - the ranking of a the number reference data sets assigned to the input measurement data set is based on the evaluation of the sum of indicator function values IFV (cf. eq. (1) or (1a)) representing a respective score of query leaves favourable for said ranking. The score of query leaves represents the number or frequency of co-occurences of reference leaves and query leaves for two given instances, i.e. the reference instance RD11 and the query instance QD. Hence the reference measurement data sets, i.e. data base samples, that occur in the same terminal nodes as the input sample will be noted and later ranked according to the frequency of co-occurrence - said score - with the input sample (lesion).

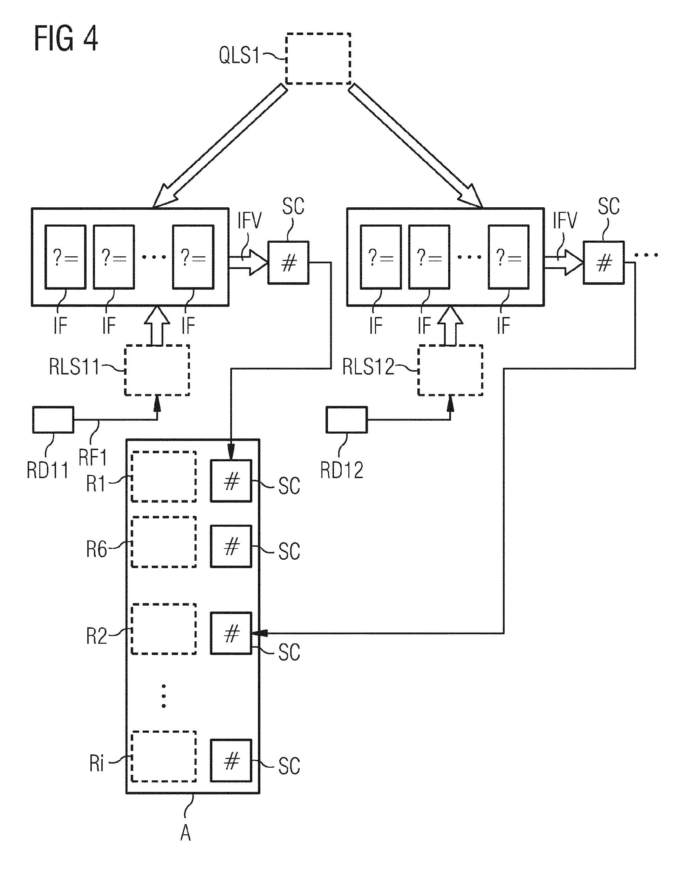

Figure 4 shows in more detail the determination of said score SC of query leaves and, accordingly, a ranking of reference measurement data sets. A query leaf set QLS1 given by the Random Forest RF1 for a query instance QD as described in view of Figure 3, is formed of k query leaves QL1, ..., QLk given by a Random Forest RF1 for the query instance QD. Analogously for each reference data set R1, ..., Ri comprised in the plurality of reference measurement data sets a reference leaf set RLS11, RLS12, ... is given by the Random Forest RF1. Each reference measurement data set R1, R2, ... is represented by a reference instance RD11, RD12, .... The sum of the indicator function values IFV, i.e. said score SC is determined by pair wise comparison of each reference leaf set RLS11, RLS12, ... with the query leaf set QLS1. For the pairwise comparison of leaf sets (i.e. the query leaf set QLS1 with the reference leaf set RLS11, RLS12, ...) the indicator function values IFV are determined pair wise for each leaf given for a particular tree comprised in the query leaf set QLS1 and a reference leaf comprised in the reference leaf set RLS11, RLS12, ... given in said same particular tree..

Accordingly, for each reference data set R1, ..., Ri (each being represented by a reference instance RD11, RD12, ...), a similarity score SC is determined, which is favourably used to provide a ranking of the reference measurement data sets R1, ..., Ri, i.e. data base samples are ranked according to said frequency of co-occurence. It is to be noted that the ranking can be provided in a variety of ways. Preferably a HTML (Hyper Text Markup Language) based list or report is generated, and a number of reference measurement data sets, denoted by A as mentioned before, are selected from the plurality of reference measurement data sets R1, ..., Ri based on comparable benignancy (context) as the lesion specified in the image section IS or ROI, comprised in the input measurement sample.

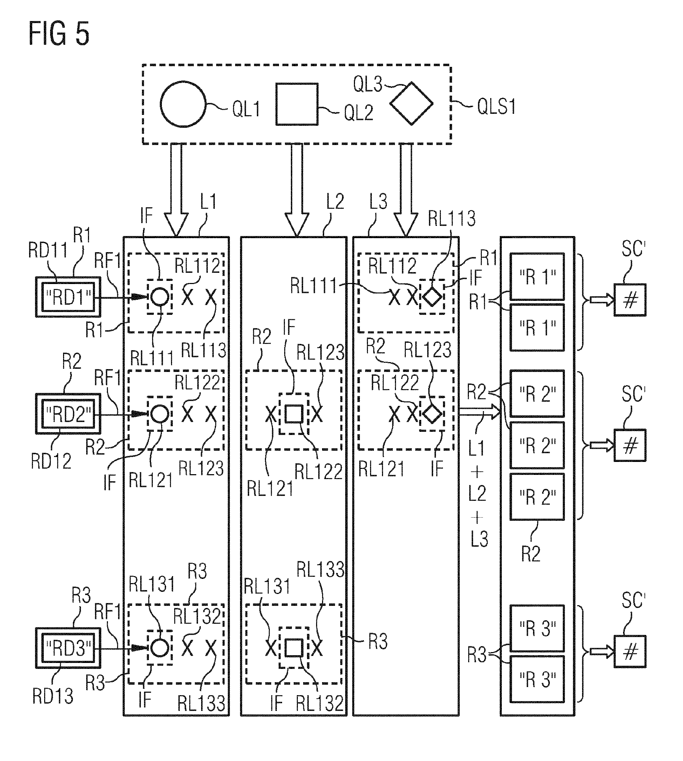

An alternative method to determine the frequency of co-occurrence or the sum of indicator function values is demonstrated in the preferred embodiment of Figure 5.

A query leaf set QLS1 as outlined in view of Figures 3 and 4 comprises query leaves QL1, QL2, QL3. It has to be noted that, for the sake of simplicity, only the first three query leaves QL1, QL2, QL3 are shown and also only three reference measurement data set R1, R2, R3. However method according to the embodiment of figure 4 can be repeated iteratively for each of the reference measurement data sets R1, ..., Ri as shown for example in Figure 3. For each particular query leaf QL1, QL2, QL3 comprised in the query leaf set QLS1 a similarity list L1, L2, L3 of reference measurement data sets R1, R2, R3 is determined, wherein for each reference measurement data set R1, R2, R3 comprised in a particular list of the similarity lists L1, L2, L3, a reference leaf RL111, R112, RL113, RL121, RL122, RL123, RL131, RL132, RL133 is given by the Random Forest RF1. This reference leaf RL111, R112, RL113, RL121, RL122, RL123, RL131, RL132, RL133 is identical to the respective query leaf QL1, QL2, QL3 for which the respective list L1, L2, L3 is determined. It has to be emphasized that the determined similarity lists L1, L2, L3 are not necessarily required in this embodiment to comprise reference measurement data sets R1, R2, R3. A list comprising identifiers, indexes or pointers which allow the identification of particular reference measurement data sets R1, R2, R3 comprised in the plurality of reference measurement data sets R1, ..., Ri (as shown in Figure 3) is also sufficient in the depicted case.

As can be further perceived, the determined similarity lists L1, L2, L3 are concatenated to a combined list LG and the number of duplicates of a particular reference measurement data set R1, R2, R3in the combined list LG is counted, reflected by score SC'. The score SC' represents said similarity score (cf. score SC presented in figure 4) or frequency of co-occurrence of leaves of two instances, and is hence a favourable measure for ranking the reference measurement data sets. Accordingly the score SC' is iteratively determined for every reference measurement data set R1, R2, R3 comprised in the combined list LG, and the reference measurement data sets R1, R2, R3 are ranked based on the score SC'.

In particular, the combined list LG is formed as follows:

For each reference measurement data set R1, R2, R3 a reference instance RD11, RD12, RD13 is determined. Preferably, reference instances RD11, RD12, RD13 are already available, for example in data base system, and can be associated to the reference measurement data set R1, R2, R3. For each reference instance RD11, RD12, RD13 reference leaves RL111, R112, RL113, RL121, RL122, RL123, RL131, RL132, RL133 are given by the Random Forest. Preferably, the reference leaves RL111, R112, RL113, RL121, RL122, RL123, RL131, RL132, RL133 determined by the Random Forest RF1 are already available, for example in a data base system, and can be associated to the associated reference instance RD11, RD12, RD13 and, accordingly, to the corresponding reference measurement data set R1, R2, R3 once they have been determined by the Random Forest. Hence frequent recalculation of reference leaves RL111, R112, RL113, RL121, RL122, RL123, RL131, RL132, RL133 for the same reference measurement data set R1, R2, R3 using the given or selected Random Forest RF1 can be obviated.

A first list L1 is attributed to a first query leaf QL1, symbolized by a circle, given in a first tree of the Random Forest RF1 for a query instance. A reference measurement data set R1, R2, R3 is included in the first list L1, in case the reference leaf RL111 given in the first tree of the Random Forest RF1 for a corresponding reference instance RD11, is identical to the first query leaf QL1; in the depicted case if the reference leaf RL111 given for the first tree is also symbolized by a circle. Hence, the determination of the list L1 is based on the pair wise comparison of reference leaves RL111, RL121, RL131, RL112, RL122, RL132, RL113, RL123, RL133 and query leaves QL1, QL2, QL3. Accordingly for each of the query leaves QL1, QL2, QL3a single, i.e. a single second, a single third, ..., a single $k^{th}$, list is determined, wherein the query leaf QL1, QL2, QL3 for which the list is determined is pair wisely compared with reference leaves RL111, RL121, RL131, RL112, RL122, RL132, RL113, RL123, RL133 associated to a respective reference measurement data set.

In the case of the preferred embodiment of Figure 5, the reference measurement data set R1 is included in the lists L1 and L3, since reference leaves associated to R1 appear pair wise with query leaves QL1 and QL3. Accordingly reference measurement data set R2 is included into the lists L1, L2 and L3, since the symbols, respectively the leaves determined for the first, second and third tree of the Random Forest are pair wisely identical to the respective query leaf. Iteratively repeating said pair wise comparison of a query leaf and a reference leaf given by the Random Forest in the same tree yields in the embodiment of Figure 5 the inclusion of a third reference measurement data set R3 in the lists L1 and L2.

Accordingly, in the present embodiment the combined list LG comprising the lists L1, L2, L3 encompasses two items referring to reference measurement data set R1, three items referring to reference measurement data set R2 and two items referring to reference measurement data set R3.

The count of duplicates in the combined list LG or score SC' is equivalent to the determined sum of indicator function values for two particular instances, and hence equivalent to the incipiently explained score SC, given by equation (1) or (1a). Accordingly said score of duplicates is a favourable criterion for ranking reference measurement data sets.

Since the clinical case data bases in hospitals are growing on a daily basis with documented cases (imaging data and related diagnostic reports), the system is ideally suited to be retrained by incorporating the new cases at a regular basis with low computational effort (as opposed to conventional batch learning).

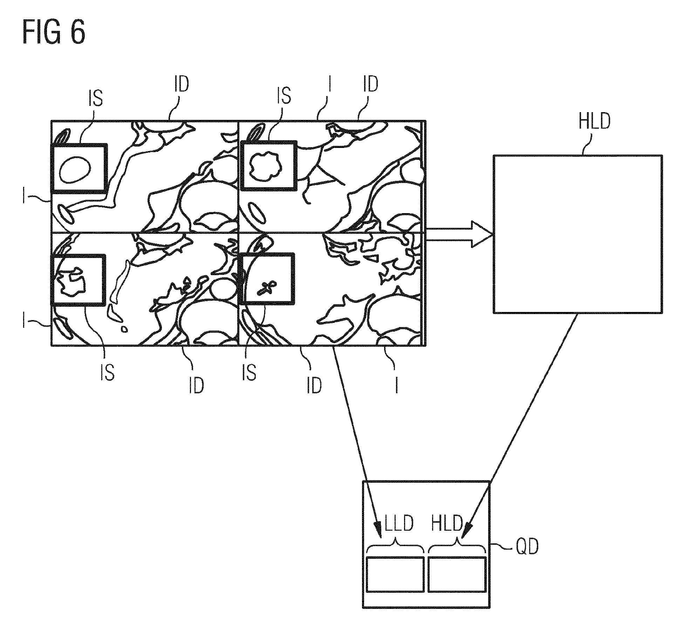

In a preferred modification of the invention as shown in Figure 6 the query instance comprises both, high level data HLD formed of high level features and low level data LLD formed of low level features.

Required semantic high-level features could be extracted from clinical reports using e.g. semantic text parsing tools to automate the training process, as will be elaborated in further detail.

Each input sample or input measurement data set for search (in our example application, each liver lesion,) is described by a set of low-level computer-generated imaging features, respectively low level data LLD. Since low-level features are computer generated, they can be calculated or counted from the image data ID, used as low level data LLD.

In the embodiment of figure 6, these are as follows:

- Relative frequency histogram and four first central normalized moments on it for Hounsfield Units (HU) distribution in the bounding box for the lesion; Hence, a low level feature comprised in the low level data LLD, is formed of a histogram of the frequency of occurrence of a certain attenuation value (Hounsfield Units) of a certain radiation intensity (X-ray intensity) determined solely in the bounding box for the lesion, respectively the image section IS. Further low level data LLD formed of low level features solely based on image data of the bounding box, may comprise the four first centralized moments formed of the expected value, the variance, the skewness and the kurtosis, calculated on said histogram.

- Four first normalized central moments on the vector of HU (Hounsfield Units) distribution for the bounding box of the lesion; Accordingly low level features comprised in the low level data LDD may be formed of the expected value, the variance, the skewness and the kurtosis calculated on the distribution of Hounsfield Units (i.e. an attenuation factor of a certain radiation density) solely based on image data ID comprised in the bounding box, i.e. an image section IS.

- Eight image moment invariants of Hu up to the 3rd order; Additionally to centralized moments known from statistics, additional low level features may be formed of centralized moments applicable in the field of image analysis. Details on image moment invariants according to Hu, that is centralized moments pertinent to image analysis, are described in M.K. Hu, Visual pattern recognition by moment in- variants, IRE Trans. Inf. Theory IT-8, 179-187 (1962).

- Six image moment invariants of Zernike up to the 3rd order; Respective details on the computation of image moment invariants according to Zernicke, pertinent to image analysis are described in Pejnovic, P., Buturovic, L.J., Stojiljkovic, Z., Object recognition by invariants,ICPR92(II:434-437).

- HU (Hounsfield Units) histogram and the four first central moments for the whole liver; respectively a low level feature is formed of the HU histogram. Additional low level features are provided by the expected value, the variance, the skewness and the kurtosis, calculated on the histogram of Hounsfield Units, i.e. the frequency of occurrence of a Hounsfield unit value of image data ID representing the whole liver, wherein the whole liver is a 3D region of a full 3D volume.

- Four first normalized central moments on the vector of HU (Hounsfield Unit) distribution for the bounding box of the whole liver.

The first feature types describe the lesion itself, while the last two describe the whole liver.

Hence the low level data LLD comprises features counted or calculated from the bounding box, i.e. an image section IS, and low level features counted or calculated from the full image data ID.

Inclusion of whole liver features, respectively features describing the full image data ID, has been shown to always lead to an improved discrimination performance in a set of experiments, as will be shown in the following.

As mentioned above, 2D Hu and Zernike invariant moments are used as low level data LLD. In order to adapt them to the 3D liver lesion boxes, for each lesion three orthogonal 2D cuts are generated, which are intersecting in the centre of the lesion. The invariant moments are then calculated for each cut, and the feature vector, respectively the query instance QD, includes both the moment for each separate cut and the averaged moments.

In the embodiment of figure 6, the query instance QD is formed of a combination of low-level data LLD and high-level data HLD. Each of the four schematically sketched CT images reflects the same liver lesion, captured for a different contrast agent phase. Although showing the identical liver section the appearance of the liver and the lesion in particular is radically diverging. Accordingly the contrast agent phase is an important feature, to describe the respective lesion. However it can not be directly counted or calculated from the input measurement sample, respectively the image data ID. Specifically important semantic high-level features could be extracted from clinical reports, or fed by other means to the query instance QD.

Advantageously each sample, i.e. the input measurement data set, (in our case, liver lesion in a 3D CT scan) is characterized with a set of semantic labels, i.e., high-level features/descriptors, in the embodiment of figure 6 including the features:

- Contrast agent phase with possible values Native, Arterial, Portal_Venous and Late;

- Lesion focality with possible values Single and Multiple;

- Lesion surrounding with possible values Complete, Incomplete and Absent;

- Rim continuity with possible values Continuous_Bright_Rim, Discontinuous_Bright_Rim, Continuous_Dark_Rim, and Discontinuous_Dark_Rim;

- Margin with possible values Regular and Irregular;

- Margin definition with possible values Defined and Diffuse;

- Lesion density with possible values Hypodense and Hyperdense; and the feature

- Benignancy with possible values Benign, Rather_Benign, Malignant and Rather_Malignant);

The query instance as shown in Figure 6 is formed of a combination of high-level data HLD and low-level data LLD, in particular presented to the system according to Figure 1 for automatic query as a vector, the so called and previously explained "feature vector", which vector represents the query instance QD.

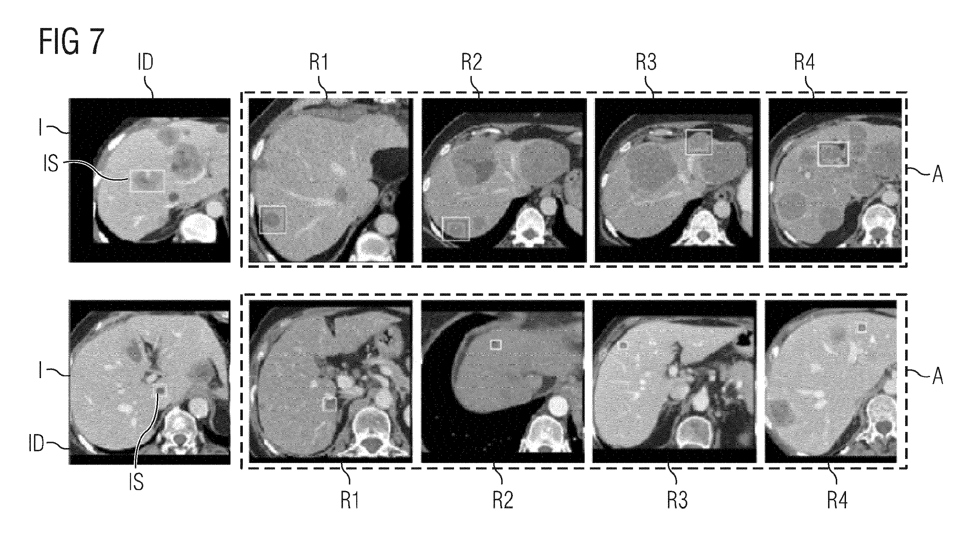

Figure 7 illustrates the top four ranked results (i.e. the assigned group of reference measurement data sets A) for two different input lesions, respectively input measurement data sets I (comprising CT image data ID with marked image section data IS), obtained within the Random Forest similarity-based framework. Figure 7 shows the results of the search for similar lesions in the benignancy similarity context. In the top row it is depicted a malignant lesion used as input (first image), and the top retrieved lesions, i.e. the assigned number of reference measurement data sets A formed of four reference measurement data sets R1,..., R4, all of which are malignant as well, even though their appearances are not necessarily comparable. In the bottom row an input benign lesion (leftmost image) and top retrieval results are shown, with all results except one being benign as well.

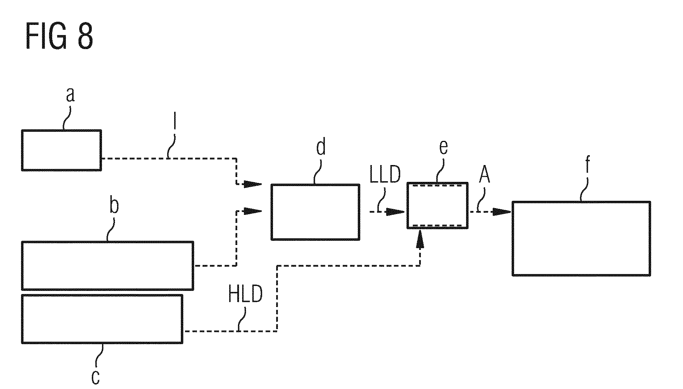

Figure 8 shows an overview of a preferred embodiment with an example application to the search of hepatic lesions with defined margin (high-level feature, representing high-level data HLD) and comparable benignancy (context) as the lesion specified in the image section, the so called "region of interest" - ROI. In Figure 8, the input sample, i.e. the input measurement data set, (provided in step a formed of the lesion with a marked ROI), is represented by both a set of low-level features (computed in step d) and by the given additional high-level features provided in step c. Based on the desired search context(i.e. the context of comparable benignancy used as search criteria provided in step b), the input sample, is run through the Random Forest trained and optimized for the benignancy context (i.e. the context selected in step b), as indicated in step e. The data base samples that occur in the same terminal nodes of the trained and selected (based on step b) Random Forest as the input sample will be noted and later ranked according to the frequency of co-occurrence with the input sample (lesion).

As can be seen from figure 8, indicated by arrows, running the input sample through the Random Forest includes the computation of low-level features in step d based on the input measurement data set (provided in step a and the context (provided in step b). Moreover running the input sample through the Random Forest also includes that the computed low-level data and the high level data is run through the Random Forest, hence, a query instance as defined before is run through the Random Forest. Furthermore, it should be noted that the Random Forest yields terminal positions for both the query instance and also a reference instances. I.e. data base samples favourably are attribute to reference instances or provide reference instances, hence, all necessary features to perform a meaningful search are comprised in the data base and are respectively assigned to a data base sample.

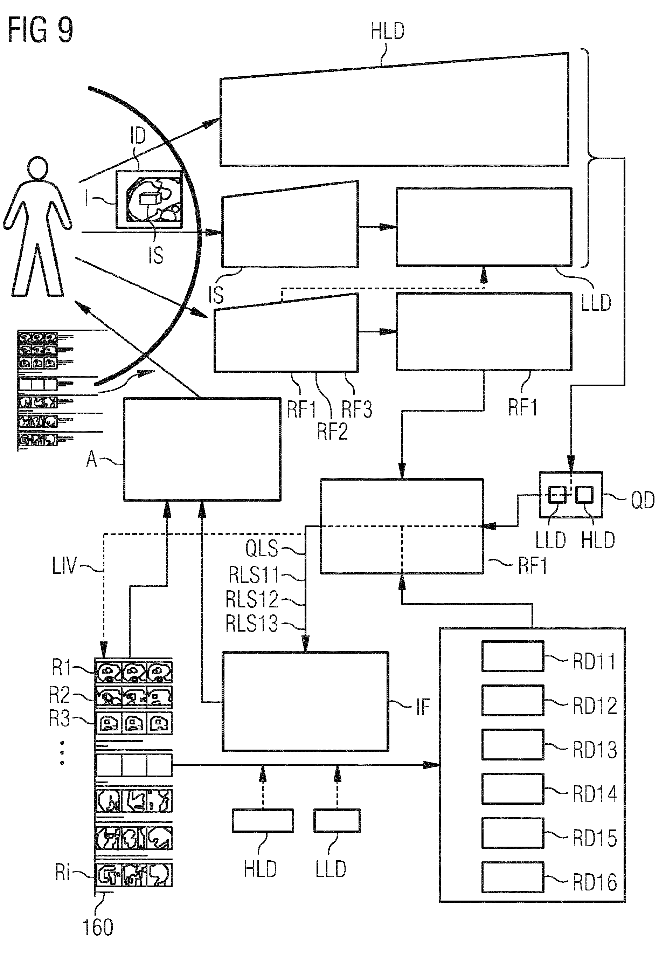

In step f the ranked list of data base samples is a ranked list of lesions based high-level features and on the desired context, in the particular embodiment of Figure 8 comparable benignancy. The ranked list is provided as a HTML report. It is included that the number of assigned measurement data sets provided in the HTML report can range from zero results (e.g. if the data base of images is empty or if there is no "similar" result according to a given threshold) to one, two, or whichever number of results is desired, limited only by the number of lesions that can be found in the data base. It is conceivable that a threshold criterion can be applied and the number of assigned reference measurement data sets is determined according to a threshold criterion. The threshold criterion can be a given similarity S (threshold similarity) or dissimilarity D (threshold dissimilarity) as defined above. However, the threshold criterion might also be a fixed number which is not to be exceeded. For instance the number of list items provided in the HTML report might be fixed to ten or below. Explicitly is to be noted that any expedient combination of the criteria mentioned above is also conceivable in the framework of the invention. Moreover, assigning more than one reference measurement data sets to the input measurement data set exhibits the benefit that additional statistical information can be extracted or gained from the assigned number of reference measurement data sets, proliferating the assessments due for the new case or image. The preferred embodiment of Figure 9 illustrates the setup sketched in Figure 8 in more detail. The present embodiment provides the possibility to retrieve a number of similar cases from a case data base, i.e. clinical images and related reports, from an electronic data base system 160 to assign a number of reference data sets A to the input measurement data set I, supporting clinical analysis of the input measurement data set I.

As sketched in Figure 9 a user of the invention, typically a physician, is due to analyze a new case, i.e. an input measurement data set I, given by a 3D CT image formed of image data ID. These image data ID include an image section IS, providing a region of interest or bounding box comprising a lesion. The assessment typically involves a variety of contexts which are to be included in the analysis for example the "benignancy of the lesion" or the "type of the lesion" is to be assessed for a comprehensive analysis. For each context, an associated Random Forest RF1, RF2, RF3 has been trained and can be selected from a plurality of available Random Forests RF1, RF2, RF3 based on a context which is chosen for analysis. Hereby, the context for an analysis is preferably identified and chosen by the physician i.e. based on high level data HLD or may also be selected automatically based on, for example, segmentation information or additional report data etc. As an arbitrary number of contexts can be applied and easily combined as search criteria in this framework, it is accordingly possible to select respectively use one or more available trained Random Forests RF1, RF2, RF3 preferably in parallel. In the embodiment of Figure 9 the Random Forest RF1 trained for the "Benignancy" context is selected and used for search. Hence assessing the similarity to data base samples yields cases with "comparable benignancy". Possible categories, i.e. terminal positions of the Random Forest, include the representation of the values "Benign", "Rather_Benign", "Malignant", "Rather_Malignant", reflecting a meaningful category for a clinical analysis of lesions.

High level data HLD based on the assessment of the new case or image, in the present embodiment provided as the respective high level feature "margin definition" with possible values "Defined" and "Diffuse" are considered in the search.

Based on the desired, i.e. selected or chosen, search context low-level features are calculated or counted based on the input measurement data set I. In the depicted embodiment the low level features are counted and/or calculated from both the full image data ID and the image section IS, as explained above. It is to be understood, that Random Forests are trained based on a set of defined features presented to the Random Forest as input preferably by incorporating cases respectively by incorporating input measurement data sets I. In case the low level data LLD are calculated or counted based on the search context, the low level data LLD are preferably comprised in the set of features which was used to train the selected Random Forest. In the present embodiment of Figure 9 the Random Forest RF1 was trained for the benignancy context based on low level data LLD including for example the feature of four first normalized central moments on the vector of HU. Accordingly the low level data calculated for the new case which is to be assessed, include the four first normalized central moments on the vector of HU.

However it is to be noted, that Random Forests are arranged to cope with heterogeneous feature vectors with possible missing values. Hence, although the Random Forest was trained relying on a set of training features, not all of these training features need to be fed as an input to the Random Forest to gain a meaningful result, proving Random Forests as particularly favourable for the present embodiment.

The combination of high level data HLD and low level data LLD forms the query instance QD which is presented and fed to the selected Random Forest RF1. The query instance QD is run through the Random Forest RF1, and the Random Forest RF1 yields query leaves, i.e. terminal positions combined and noted as a query leaf set QLS for the query instance QD.

In order to assess similarity, data base samples, i.e. a plurality of reference measurement data sets R1, R2, R3,..., are provided in a data base system 160. As the data base samples are to be categorized for the assessment of similar benignancy by the selected Random Forest RF1, the features presented to the Random Forest as input need to be at least partially relevant for the desired context. Accordingly, for each of the plurality of measurement data sets suitable features are provided, comprising both high level data HLD and low level data LLD attributed to that context. In Figure 9 the high level feature assigned to the data base samples (reference measurement data sets R1, ..., Ri) is "margin definition", represented by the values "Defined" or "Diffuse". Accordingly the high level feature as used for the query instance QD is also used to describe reference measurement data sets R1, ..., Ri. However, said coincidence is not mandatory. As mentioned before, Random Forests are arranged to cope with heterogeneous feature vectors with possible missing values.

The combination of high level data HLD and low level data LLD provided for each specific reference measurement data set R1, ..., Ri forms a reference instance RD11, RD12, RD13, ... presented to the selected Random Forest RF1.

Accordingly, the reference instances RD11, RD12, RD13, ... are run through the selected Random Forest RF1. For each reference instance RD11, RD12, RD13, ... a reference leaf set RLS11, RLS12, RLS13, ... is given by the Random Forest RF1, each providing a set of noted and preferably combined terminal positions for the respective reference instance RD11, RD12, RD13,....

Similarity is assessed as elaborated before, based on the pair wise evaluation of the indicator function IF for a query leaf comprised in the query leaf set QLS and a reference leaf comprised in a reference leaf set RLS11, RLS12, RLS13, ... which are given in the same tree by the Random Forest (cf. equation (1)). The similarity score formed of the sum of indicator function values is determined for two sets of leaves, the query leaf set QLS and a reference leaf set RLS11, RLS12, .... As elucidated before each leaf of one of said leaf sets QLS, RLS11, RLS12, RLS13, ... in which the leaf is given for a particular tree of the Random Forest, is pair wisely compared via the indicator function IF with another leaf (which is) given in the same particular tree. The other leaf is comprised in the other one of said leaf sets QLS, RLS11, RLS12, RLS13, .... As also previously indicated, the score and hence the similarity of cases provided in the data base to the new case, which is due to be assessed, provides a ranking of data base samples R1, R2, R3. Related or assigned to the new case a ranked list (i.e. the group of assigned reference measurement data sets A) of data base samples is generated and provided to the user, based on the determined similarity. In order to avoid a frequent recalculation of reference leaves a leaf index vector LIV, reflecting the tree and leaf information (i.e. the information that relates a leaf or category to a tree) of a reference leaf set RLS11 in a predetermined order, is stored in the data base system 160 and is assigned to the respective reference measurement set R1. Furthermore, the data base system 160 is realized to provide the leaf index vector LIV preferably on request, advantageously in combination with a related reference measurement data set R1, R2, R3.

As already mentioned before, embodiments of the invention can be arranged to apply an arbitrary number of contexts which can be easily combined as search criteria.

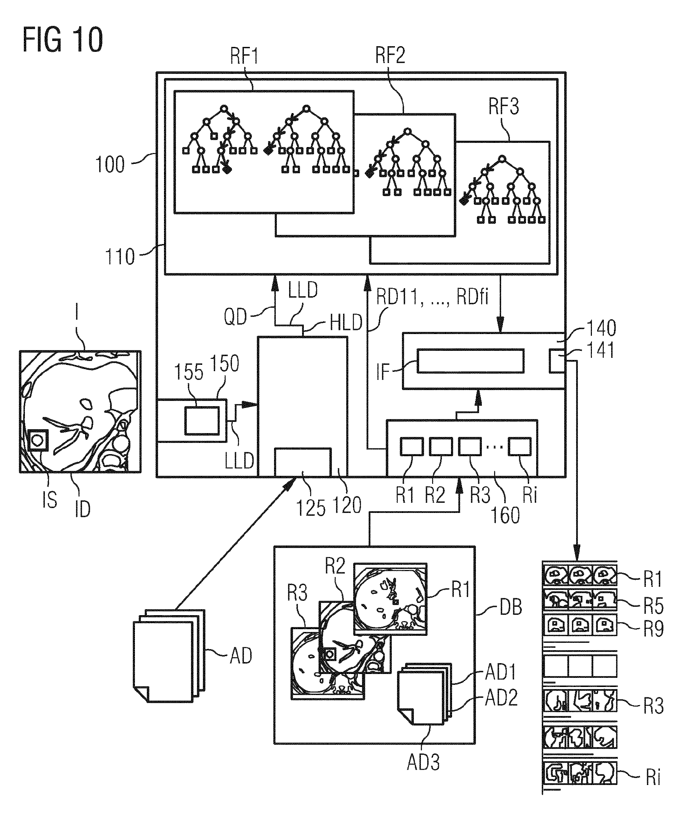

In an embodiment of the invention as shown in Figure 10 the selection between multiple contexts and also the possibility to combine multiple contexts is described. Figure 10 displays an embodiment of a system 100 for assigning a number of reference measurement data sets R1, R2, ... selected from a plurality of reference measurement data sets R1, R2, R3, ... to an input measurement data set, realizing a CBIR system as explained using Figure 1. The majority or all components of the system 100 can be realized as software components.

The system 100 comprises an input unit 150 for providing a new case I respectively image data ID to other members of the system 100. As is also shown in figure 10, the input unit 150 is formed to receive image data ID comprising a marked region of interest, or respectively a marked image section IS.

In the embodiment of figure 10 the input unit 150 comprises a low-level calculation unit 155 realized to calculate low-level features and to provide the low level features as low level data LLD to the query unit 120.

However, it is conceivable that the low-level calculation unit 155 is arranged independently of the input unit 150. The term "arranged independently" includes both arranging data bases internally and external to the system 100. In case the low-level calculation unit 155 is arranged external to the system 100, it is conceivable that the input unit 150 is realized to receive low-level data LLD, calculated by the externally arranged low-level calculation unit 155.

Alternatively or additionally, the low-level calculation unit 155 may be arranged as a member of the query unit 120, which is also comprised in the system 100, realized to determine a query instance QD based on the new case I, i.e. based on low level data LLD and, as elucidated below in more detail, high level data HLD.

As explained, said high level data can be preferably extracted from medical reports, and hence the system 100 comprises a suitable high level input interface 125 for recognizing and/or parsing additional data AD. In the depicted embodiment the high level input interface 125 is arranged as a member of the query unit 120 and is favourably realized to parse texts, in particular texts of clinical reports.

Alternatively or in addition, the high level input interface 125 can be arranged anywhere internal or external to the system 100 and in particular as a member of the input unit 150. Hence new cases can be presented to and acquired by the system 100 as a combined file of image data ID and additional data AD, wherein the acquired data can be fed to the other members of the system 100 by the input unit 150.

The high-level input interface 125 in the embodiment of Figure 10 comprises a scanner to digitize reports available on paper, and respective parsing units to parse medical reports for key words and/or markers which preferably can be given or fed to the text parsing units or selected based on a specific context. Hence the text parsing unit is realized to identify given key words based on a selected context.

The system 100 comprises further a data base system 160, designed to provide a plurality of reference measurement data sets R1, ..., Ri. The data base system 160 includes one or more memories, in the present case both a persistent memory, as for example a magnetic memory (including hard disc drives and the like), an optical memory, a flash memory or the like, and possibly also a volatile memory as for example a DRAM. The memories and also a link to an external data base system DB foster the ability to provide a plurality of reference measurement data sets R1, ..., Ri to the system 100. As shown in Figure 10 the external data base system DB comprises additional support data sets AD1, AD2, AD3 represented by medical reports comprising annotations of lesions to be analyzed. The system 100 is arranged to retrieve or respectively access the combination of reference measurement data sets R1, R2, R3 and medical reports, i.e. additional support data sets AD1, AD2, AD3 as a combined data set or file, respectively as a medical "case" formed of image data and the related annotation or the medical report.

Furthermore, the system 100 comprises a Random Forest categorization unit 110 for providing and selecting one ore more Random Forests RF1, RF2, RF3 arranged for categorization of the input measurement data set I, as explained above. Favourably, the reference measurement data sets R1, ..., Ri comprised in the data base system 160 can also be categorized based on reference instances RD11, ..., RDfi as hinted by a respective arrow. Here again, index f points to a particular Random Forest RF1, RF2, RF3 and index i identifies a specific reference measurement data set R1, ..., Ri. The reference instances RD11, ..., RDfi are preferably stored in one or more of the data base systems 160, DB. The reference instances RD11, ..., RDfi have been determined by the low level calculation unit 155 or a similar unit comprised in the system 100 and/or a parsing unit to process additional support data AD1, AD2, AD3, which are related to the respective reference measurement data set R1, R2, R3. Preferably the parsing unit is one of the previously described parsing units. Accordingly, the reference instance R1, ..., Ri can be determined by the query unit 120.

As can be readily perceived from Figure 10, the random forest categorization unit 120 provides multiple Random Forests RF1, RF2, RF3 each trained for a single particular context as for example "benignancy" or "lesion type". The random forest categorization unit 110 determines query leaves of the Random Forest RF1, RF2, RF3 based on the query instance QD, i.e. based on high and low level data HLD, LLD associated to or describing the input measurement data set I, hence the new case.

In the same way, the reference measurement data sets R1, ..., Ri are categorized by one or more of the random forests RF1, RF2, RF3.

Even more preferably, the reference measurement data sets R1, ..., Ri in the data base system 160 and/or the external data base system DB have been already categorized by one or more of the available Random Forests RF1, RF2, RF3 and the categorization result, i.e. the leaves determined or given by the Random Forest RF1, RF2, RF3 associated to respective reference measurement data sets R1, ..., Ri are retrievable and hence are stored in the internal data base system 160 and/or the external data base system DB.

The random forest categorization unit 110 also comprises one or more memories, into which Random Forests RF1, RF2, RF3 can be loaded single and/or in parallel for categorization of data sets. The categorization unit 110 is realized to select between and access an arbitrary number of Random Forests RF1, RF2, RF3. Hence, an arbitrary number of Random Forests RF1, RF2, RF3 can be loaded into the memory before categorization and the categorization result (i.e. query leaves and or reference leaves) is held in the respective memory.

Furthermore, the system 100 comprises a similarity evaluation unit 140. The similarity evaluation unit determines the similarity score of query leaves to respective reference leaves determined by the Random Forests RF1, RF2, RF3 for the reference instances RD11, ..., RDfi based on the indicator function IF as previously elucidated.

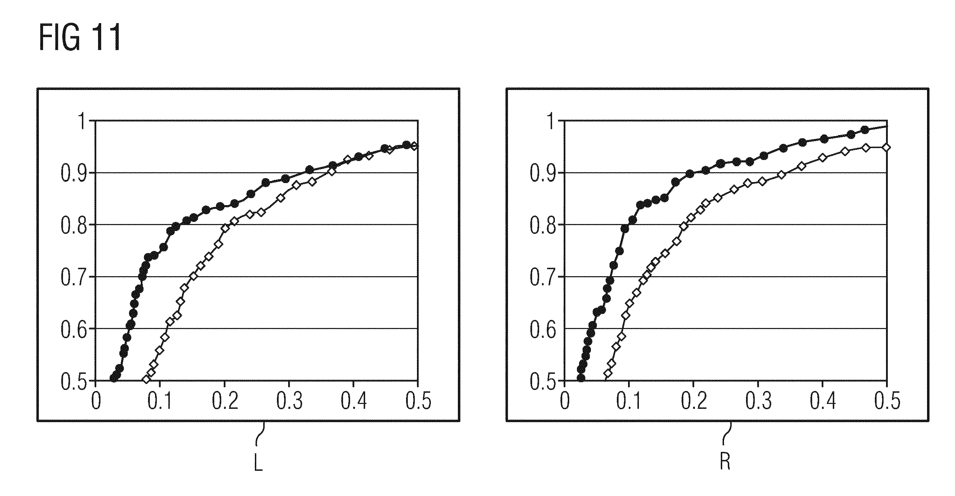

In the present embodiment of figure 11 the reference leaf sets are stored in the data base system 160 as mentioned before as a leaf index vector, related to both Random Forests RF1, RF2, RF3 and reference measurement data sets R1, ..., Ri, respectively to reference instances RD11, ..., RDfi

Furthermore the similarity evaluation unit 140 determines and assigns a number of reference measurement data sets A to the input measurement data set I, based on the determined similarity score as elucidated previously. Favourably, the similarity evaluation unit 140 comprises input means, as for example a data interface, a terminal interface or any other user interface to select reference measurement data sets R1, R2, R3 comprised in the number or group of assigned reference measurement data sets A for retrieval, and to select the maximum number of reference measurement data sets R1, R2, R3, retrieved and assigned to the input measurement data set I. Furthermore, via the user or data interface, also a number of statistical evaluations are selectable, based on the determined group of reference measurement data sets A.

In particular, a differential diagnosis together with a confidence probability can be determined compiled by the similarity evaluation unit 140.

Statistical evaluations and the compilation of a differential diagnosis include for example the computation and display of distribution histograms for clinical variables (variables which may be tested, like for example blood test values) of the retrieved cases.

Accordingly the system 100 is arranged to provide machine readable additional support AD data, and the system 100 is realized to perform additional counts or calculations on the machine readable support data.

Said evaluations are performed in the present embodiment in the similarity evaluation unit 140. However, it is also conceivable that the number of assigned reference measurement data sets A are accompanied with a trigger information, which can be assessed by other systems to perform a appropriate post processing of the retrieved reference measurement data sets A.

Furthermore, the similarity evaluation unit 140 comprises an output interface 141 to display, export, distribute or combine the number of assigned reference measurement data sets A to or as a preferably ranked list of reference measurement data sets R1, ..., Ri and additional support data AD1, AD2, AD3, in the present case combined as a HTML report, comprising both 3D CT image data and respective annotations for the images. Based on said list and the retrieved additional information an evidence based assessment of the input measurement data set I can be performed by the physician. It is to be pointed out, that providing the list as HTML data provides the possibility to assess the data with standard software independent of the system 100, fostering a variety of post processing options.

Figure 11 illustrates the retrieval accuracy gained for specific embodiments of the invention and hence provides a measure for the support of the physician provided by the invention. For hepatic lesion retrieval, two search contexts have been tested; lesion benignancy and (lesion) type. For each context, an associated Random Forest has been trained with the following annotated lesions:

- Benignancy (Benign, Malignant): 761 malignant and 93 benign lesions.
- Type (Cyst, Metastasis): 1103 metastases and 98 cysts.

The preferred embodiments of the invention are flexible enough to accommodate any combination of high-level and low-level features, and have been tested in the two aforementioned contexts in a Leave-One-Patient-Out (LOPO) evaluation. The assessed retrieval results were as follows:

The retrieval performance in an embodiment of the invention using the two specified contexts, in terms of discrimination accuracy of the Random Forest-based distance learnt, is shown in the following. We use LOPO ROC AUC (Area Under the Curve) values to evaluate the ultimate performance. A LOPO (Leave One Patient Out) evaluation is a cross-validation in which, for each patient, images corresponding to the single patient are left out and used as a test input measurement data set to verify the accuracy of the categorization and the remaining rest of images is used as the training input measurement data set. Then the results are averaged over the patients or a representative ROC (Receiver Operating Characteristic) curve can be obtained. A single commonly used measure to characterize an ROC is AUC, respectively "Area Under the Curve".

For each of the two search criteria (i.e. contexts or Random Forests) and each representation of the input sample (ROI), the tables below show the ROC AUC measure of the retrieval results. The ROC curves pertaining to the training of the Random Forests in the two different contexts are shown in Figure 11.

Figure 11 depicts in a left diagram L and a right diagram R ROCs for the training of the RFs for each context. From left to right, Benignancy and Type context ROCs of the corresponding trained classifiers (i.e. categories represented by leaves of the Random Forest). In each case, the line marked with diamonds shows the curve with low-level features only, while the line marked with dots shows the curve when additional high-level features are included as input, respectively derived from annotations or other additional supplementary data sets.

An overview of related experimental data is given in the following tables:

| | All lesion sizes | | |
|---|---|---|---|
| Context | low level features | low and high level features | Gain |
| Benignancy | 0.854 | 0.891 | 0.037 |
| Type | 0.0872 | 0.906 | 0.034 |
| Average | 0.863 | 0.8985 | 0.0355 |
| | Small lesions (<10x10x10mm) | | |
| Context | low level features | low and high level features | Gain |
| Benignancy | 0.732 | 0.855 | 0.123 |
| Type | 0.755 | 0.838 | 0.083 |
| Average | 0.7435 | 0.8465 | 0.103 |

The tables above provide a quantitative evaluation of retrieval results in the two contexts: Lesion Benignancy and Type using, in each case, low-level features alone or a combination of low- and high-level features. The tables shows the results for all lesion sizes combined, and sub-centimeter lesions are studied separately.

It is interesting to observe that when high-level clinical contexts such as benignancy or lesion type are considered, the positive impact of the additional high-level features or high level data, given as input, is clear and results in better accuracy and more relevant retrieval results. This benefit is particularly visible in the case of sub-centimeter lesions, which are often very hard to characterize even for the expert eye. In this case, the addition of one or more high-level features has a significant influence on the accuracy and meaningfulness of the results. In a clinical context where small lesions are difficult to assess, embodiments of the invention have great potential to become a significant decision support tool. An arbitrary number of contexts can be applied and easily combined as search criteria in the framework of the invention.

Based on the invention on one hand, a fast distance learning-based CBIR framework based on the intrinsic Random Forest similarity is introduced. The system can be trained and optimized for a given clinical context.

Based on the invention, on the other hand, the combination of flexible user-defined contexts and high-level concepts with low-level features is allowed in order to yield search results that are both meaningful to the user and relevant in the given context.

The invention has been implemented and tested in an embodiment arranged for liver lesion retrieval. The application of the invention described in this disclosure resulted in an accuracy improvement of over 3% for lesions of all sizes and over 10% for small (i.e. particularly complicated) lesions with regards to the original (low-level feature only search criteria) approach.

The proposed invention is ideally suited to be used as decision support system together with existing diagnostic reading applications Given a new case for which a differential diagnostis or therapy planning needs to be done, the invention supports decision making in two aspects:

Firstly, the system can be trained for a specific differential diagnosis task and outputs a differential diagnosis together with a confidence probability, e.g. that an unkown lesion is a cyst or metastasis.

Secondly, the system is realized to retrieve a ranked list of patients from the case data base that are most "similar" to the given patient where "similarity" corresponds to the specifically-learned clinical context. Those similar cases together with their documented differential diagnosis and course of therapy provide additional valuable information for the decision making about the newly given patient being examined (Case-based reasoning (CBR)).

Although the present invention has been disclosed in the form of preferred embodiments and variations thereon, it will be understood that numerous additional modifications and variations could be made thereto without departing from the scope of the invention. For the sake of clarity, it is to be understood that the use of "a" or "an" throughout this application does not exclude a plurality, and "comprising" does not exclude other steps or elements. The mention of a "unit" does not preclude the use of more than one unit.

**Claims**

1.  Method for assigning a number of reference measurement data sets (A, R1, R2, R3, R4, R5, R7, R9), selected from a plurality of reference measurement data sets (R1, ..., Ri) to an input measurement data set (I) comprising the following steps:

    - determining a query instance (QD) based on the input measurement data set (I),
    - providing a Random Forest (RF1, RF2, RF3),
    - determining query leaves (QL1, ..., QLk) of the Random Forest (RF1, RF2, RF3) based on the query instance (QD),
    - assigning the number of reference measurement data sets (A, R1, R2, R3, R4, R5, R7, R9) to the input measurement data set (I), based on the determined query leaves (QL1, ..., QLk) and based on reference leaves (RL111, RL112, ..., RLfik) determined by the Random Forest (RF1, RF2, RF3)) for a plurality of reference instances (RD11, ..., RDfi) based on the plurality of the reference measurement data sets (R1, ..., Ri)).

2.  Method according to claim 1, **characterized in that** the assigning of the number of reference measurement datasets (A, R1, R2, R3, R4, R5, R7, R9) to the input measurement dataset (I) is based on an indicator function value (IFV), which represents whether a query leaf (QL1, ..., QLk) based on the query instance (QD) appears together with a reference leaf (RL111, RL112, ..., RLfik) based on a reference instance (RD11, ..., RDfi).

3.  Method according to claim 2, **characterized in that** a similarity score (SC) of query leaves (QL1, ..., QLk) of the query instance (QD) which appear together with a reference leaf (RL111, RL112, ..., RLfik) of a reference instance (RD11, ..., RDfi) is determined for each of the plurality of reference instances (RD11, ..., RDfi) and the plurality of reference data sets (R1, ..., Ri) is ranked according to said determined score (SC).

4. Method according to one of the claims 1 to 3, **characterized in that**, the number of reference measurement data sets (A, R1, R2, R3, R4, R5, R7, R9) is assigned based on the portion of the trees of the Random Forest, wherein query leaves (QL1, ..., QLk) based on a query instance (QD) appear together with reference leaves (RL111, RL112, ..., RLfik) based on a reference instance (RD11, ..., RDfi), determined for each reference measurement data set (R1, ..., Ri) comprised in the plurality of reference measurement data sets (R1, ..., Ri).

5. Method according to one of the claims 1 to 4, **characterized in that** the input measurement data set (I) comprises medical image data (ID).

6. Method according to claim 5, **characterized in that** the query instance (QD) comprises features counted or calculated from image section data (IS) and/or from the full image data (ID).

7. Method according to claim 6, **characterized in that**, the query instance (QD) comprises a set of low level data (LLD), wherein low level data (LLD) is formed of low level features that can be counted or calculated from the input measurement data set (I).

8. Method according to claim 7, **characterized in that** low level data (LLD) based on an input measurement dataset (I) comprising image data (ID) is selected from a group of

- relative frequency histograms counted or calculated from image section data (IS) and/or
- four central moments calculated on the relative frequency histogram and/or
- four first normalized central moments based on an intensity histogram counted or calculated from image section data (IS),
- eight image moment invariants according to HU up to the $3^{rd}$ order calculated from image section data, and/or
- six image moment invariants according to Zernike up to the $3^{rd}$ order calculated from image section data, and/or
- an intensity histogram of the full image data (ID), and/or
- four first normalized central moments calculated based an intensity histogram counted or calculated from the full image data (ID).

9. Method for assigning a reference measurement data set (RF1, RF2, RF3) selected from a plurality of reference measurement data sets (R1, ..., Ri) to an input measurement data set (I) comprising medical image data (ID), preferably according to one of the claims 5 to 8, comprising the following steps:

- determining a query instance (QD) comprising a set of high level data (HLD), wherein high level data (HLD) are formed of high level features, distinct from low level data (LLD) and high level features are chosen from a set of predefined features.
- providing a Random Forest (RF1, RF2, RF3)
- determining query leaves (QL1, ..., QLk) of the Random Forest (RF1, RF2, RF3) based on the query instance (QD),
- assigning a reference measurement data set (R1, ..., Ri)to the input measurement data set (I), based on the determined query leaves (QL1, ..., QLk) and based on reference leaves (RL111, RL112, ..., RLfik) determined by the Random Forest (RF1, RF2, RF3) for a plurality of reference instances (RD11, ..., RDfi) based on the plurality of reference measurement data sets (R1, ..., Ri).

10. Method according to claim 9, **characterized in that** high level data (HLD) based on an input measurement dataset (I), which is formed of image data (ID) is selected from a group of

- Contrast agent phase,
- Lesion focality,
- Lesion surrounding,
- Rim Contiunity,
- Margin.

11. Method according to one of the claims 1 to 10, **characterized in that** the method further comprises selecting the Random Forest (RF1, RF2, RF3) from a plurality of Random Forests ((RF1, RF2, RF3) and preferably providing the query instance (QD) based on the selected Random Forest.

12. Method according to one of the claims 1 to 11, **characterized in that** leaves determined by the Random Forest are provided as a set of leaves (QLS1, RLS11, ..., RLSfi), wherein a leaf index vector (LIV) is formed by said set of leaves (QLS1, RLS11, ..., RLSfi) and is assigned to measurement data sets ((R1, ..., Ri) comprised in the data base (160, DB), wherein preferably reference data sets ((R1, ..., Ri) comprised a the data base (160, DB)) are indexed according to said provided set of leaves (RLS11, ..., RLSfi), wherein preferably the data base (160, DB) is arranged to assign a plurality of sets of leaves (RLS11, ..., RLSfi) to the reference data sets (R1, ..., Ri).

13. Method according to one of the claims 1 to 12, **characterized in that** input measurement data set (I) is categorized according to the determined query leaves (QL1, ..., QLk).

14. Method according to one of the claims 1 to 12, wherein the provided Random Forest (R1, ..., Ri) is re-trained in predetermined intervals.

15. System (100) for assigning a number of reference measurement data sets (A, R1, R2, R3, R4, R5, R7, R9) selected from a plurality of reference measurement data sets (R1, ..., Ri) to an input measurement data set (I) comprising

- a query unit (120) for determining a query instance (QD) based on the input measurement data set (I),
- a data base system ((160) for providing a plurality of reference measurement data sets (R1, ..., Ri),
- a Random Forest categorization unit (110) for providing a Random Forest (RF1, RF2, RF3) and determining query leaves (QL1, ..., QLk) of the Random Forest (R1, ..., Ri), based on the query instance (QD),
- and an evaluation unit (140) for assigning the number of reference measurement data sets (A, R1, R2, R3, R4, R5, R7, R9) to the input measurement data set (I) based on the determined query leaves (QL1, ..., QLk) and based on reference leaves (RL111, RL112, ..., RLfik) determined by the Random Forest (RF1, RF2, RF3) for the plurality of the reference measurement data sets (R1, ..., Ri).

16. System (100) for assigning a reference measurement data set ((R1, ..., Ri)) selected from a plurality of reference measurement data sets (R1, ...,Ri) to an input measurement data set (I), preferably according to claim 15, comprising

- query unit (120) for determining a query instance(QD) based on the input measurement data set (I), wherein the query instance comprises a set of high level data (HLD) and a set of low level data (HLD), - a data base system (160) for providing a plurality of reference measurement data sets (R1, ..., Ri),
- a Random Forest categorization unit (110) for providing a Random Forest (RF1, RF2, RF3) and determining query leaves (QL1, ..., QLk) of the Random Forest (RF1, RF2, RF3), based on the query instance (QD),
- and an evaluation unit (140) for assigning a reference measurement data set (R1, ..., Ri)to the input measurement data set (I), based on the determined query leaves (QL1, ..., QLk) and based on reference leaves (RL111, RL112, ..., RLfik) determined by the Random Forest (RF1, RF2, RF3) for the plurality of the reference measurement data sets (R1, ..., Ri) based on a plurality of reference instances (RD11, ..., RDfi).

FIG 1

FIG 2

# FIG 3

FIG 4

# FIG 5

FIG 6

FIG 7

FIG 8

a

l

b

c

d

LLD

e

A

f

HLD

FIG 9

FIG 10

FIG 11

FIG 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BREIMAN L.** Random Forests. *Machine Learning,* 2001, vol. 45 (1), 5-32